# EUROPEAN PATENT APPLICATION

(11) **EP 1 790 301 A1**
(43) Date of publication of application: **30.05.2007**
(21) Application number: 04807985.9
(22) Date of filing: 28.12.2004
(51) Int. Cl.: A61B 17/56, A61B 17/58, A61F 2/44, A61L 27/00

(54) **ARTIFICIAL INTERVERTEBRAL DISK INSERTION JIG AND JIG SET, AND ARTIFICIAL INTERVERTEBRAL DISK**

(30) Priority: 31.08.2004 JP 2004252147
(71) Applicant: TAKIRON CO., LTD., Osaka-shi, Osaka 541-0052 (JP)
(72) Inventor: SHIKINAMI, Yasuo, c/o Takiron Co., Ltd., Osaka-shi, Osaka 5410052 (JP); TSUTA, Kaoru, c/o Takiron Co., Ltd., Osaka-shi, Osaka 5410052 (JP); KAWABE, Yasuhiro, c/o Takiron Co., Ltd., Osaka-shi, Osaka 5410052 (JP)
(74) Representative: HOFFMANN EITLE
(86) International application number: PCT/JP2004/019630
(87) International publication number: WO 2006/025125

(57) **Abstract**

Subjects are to provide a stand-alone artificial intervertebral disk which has flexible, nearly ideal, mechanical deformation properties and durability and is capable of being bonded and fixed to vertebral bodies at a high force and to provide an artificial-intervertebral-disk insertion jig with which the artificial intervertebral disk can be easily inserted and disposed between vertebrae without fail.

The artificial intervertebral disk has a constitution comprising: a core material comprising a structure which is either a multiaxis three-dimensional woven structure or knit structure made of organic fibers arranged along three or more axes or a structure comprising a combination of the woven structure and the knit structure; and plates superposed respectively on the upper and lower sides of the core material, the plates being made of a biodegradable and bioabsorbable polymer containing bioactive bioceramic particles. Preferably, the plates have many perforations formed therein or the plates are ones in which the perforations are filled with a biodegradable and bioabsorbable material having bone conductivity and/or bone inductivity and having a high degradation rate. The insertion jig has a constitution comprising an upper jig and a lower jig, as a pair of jigs, which have been fitted to each other so as not to separate from each other in vertical directions and as to be capable of sliding against each other in back-and-forth directions, the upper jig and the lower jig having an upper holding part and a lower holding part which serve to hold an artificial intervertebral disk and have been formed as front parts of the upper jig and lower jig.

## Description

### FIELD OF THE INVENTION

The present invention relates to a stand-alone, biomimetic, artificial intervertebral disk having a constitution including as a core material a fibrous structure comprising a three-dimensional woven material, an artificial-intervertebral-disk insertion jig for easily inserting this artificial intervertebral disk between adjacent vertebral bodies and fixing it in the right position, and a jig set comprising this artificial-intervertebral-disk insertion jig and an insertion guide jig.

### BACKGROUND ART

Various artificial intervertebral disks have been developed. Among the useful ones is an artificial intervertebral disk of a sandwich structure which comprises an upper and lower metallic plate made of, e.g., a titanium alloy and, disposed between the plates, spheres of ultrahigh-molecular polyethylene which are intended to function as a ball bearing and impart the movability required of intervertebral disks. Although the movabiilty of this artificial intervertebral disk permits the upper and lower vertebral bodies to move, the dynamic behavior of this artificial intervertebral disk considerably differs from that of intervertebral disks of the living body. In addition, since this kind of artificial intervertebral disk is of the whole replacement type, it is necessary to insert it from the front side by a complicated operation technique and it is impossible to insert it from the back side, although insertion from the back side can be conducted by a simple operation. Because this artificial intervertebral disk is held with a special jig such as pliers and inserted between vertebrae from the front side (venter side) of the vertebral column, the operation is elaborate and an unskilled doctor cannot easily perform the operation. There is hence a serious problem that the insertion of this prior-art artificial intervertebral disk is contrary to the current trend toward low-invasion operation techniques.

As another method of treatment for an intervertebral disk damage, the fusion cage method in which a cage is used in order to fusion-fix adjacent vertebral bodies (bones) to each other has come to be practically used with various materials [e.g., ones made of allograft bone, stainless steel, titanium, carbon, or PEEK (poly ether ether ketone)]. For example, a hollow fusion cage of a nearly rectangular-parallelepiped shape with an opening has been proposed which has, in the rear wall part, a screw hole into which the front end of a rod-form positioning tool (insertion jig) is to be screwed (patent document 1). This fusion cage is inserted between adjacent vertebral bodies from the back side of the vertebral column after the front end of the rod-form tool is screwed into the screw hole in the rear wall part of the fusion cage. Consequently, the operation is simple as compared with the case of the sandwich-structure artificial intervertebral disk described above, which is to be inserted between vertebral bodies from the venter side.
However, this fusion cage proposed in patent document 1 is intended not to enable the patient to recover the same movability as that of vertebral disks of the living body, but to be used in an operation in which the adjacent vertebral bodies are fixed, after the insertion of the cage, with an auxiliary device for fixing (screw or plate) or the like so as to eliminate movement. This operation technique is hence not a treatment of intervertebral disks which is truly desired by the doctor and patient.

Under these circumstances, the present applicant proposed a biomaterial for use as an artificial cartilage, such as, e.g., a stand-alone type artificial intervertebral disk (patent document 2). This biomaterial comprises: a core material comprising a three-dimensional fibrous structure which is either a multiaxis three-dimensional woven structure or knit structure made of organic fibers arranged along three or more axes or a structure comprising a combination of these; and spacers respectively superposed on and united with both sides of the core material, the spacers having interconnected pores and comprising a porous object of a biodegradable and bioabsorbable polymer containing bioactive bioceramic particles. The artificial intervertebral disk comprising this biomaterial can effectively function as a substitute for an intervertebral disk of the living body because the core material comprising the fibrous structure has almost the same mechanical flexibility (movability) as normal intervertebral disks of the living body and the deformation properties thereof are highly biomimetic and because the spacers superposed directly bond to the upper and lower vertebral bodies and are replaced by bone tissues with the lapse of time to thereby fix the surfaces of the fibrous structure to the upper and lower vertebral bodies.

The artificial intervertebral disk described above is exceedingly effective in bonding to vertebral bodies because the spacers have excellent bone conductivity orbone inductivity. However, there is a fear that the spacers may deform due to compression by weight with the penetration of bone tissues into the spacers and the growth thereof. There is hence a possibility that the replacement of the spacers by bone tissues and the bonding between vertebral bones and the artificial intervertebral disk might become incomplete, resulting in a lowered force of bonding/fixing to the upper and lower vertebral bodies. Furthermore, the spacers comprising a porous object are brittle and, hence, there also has been a possibility that the peripheries of the spacers wear to generate fine particles. In addition, since the core material of this artificial intervertebral disk is a three-dimensional fibrous structure, the technique in which a screw hole is formed and the front end of a rod-form insertion jig is screwed into the hole in preparation for insertion, as in the case of the fusion cage of patent document 1, cannot be used for inserting the artificial intervertebral disk between vertebrae. It has hence been necessary to immediately develop a partial replacement type artificial intervertebral disk employing a three-dimensional fibrous structure as a core material and an insertion jig which are suitable for easily inserting the artificial intervertebral disk between vertebrae without fail from the back side of the vertebral column. Furthermore, with respect to a whole replacement type artificial intervertebral disk which can be inserted between vertebrae by a relatively easy operation, as in cervical vertebrae, through the incision of a part located on the front side of the cervical vertebral column and also to a whole replacement type artificial intervertebral disk for lumbar vertebrae, it has been necessary to immediately develop a jig which is suitable for the insertion of these artificial intervertebral disks from the front side.
Patent Document 1: JP-A-2004-195232
Patent Document 2: JP-A-2003-230583

### DISCLOSURE OF THE INVENTION

The invention has been achieved under the circumstances described above. Subjects for the invention are: to provide an artificial intervertebral disk which includes a fibrous structure as a core material, has flexible and nearly ideal deformation properties, can be bonded to vertebral bodies without fail, and can be fixed at a high force; and to provide an artificial-intervertebral-disk insertion jig which is excellent in controllability and handleability and with which the artificial intervertebral disk can be easily inserted between vertebrae without fail. Another subject is to provide a jig set comprising this artificial-intervertebral-disk insertion jig and an insertion guide jig.

In order to eliminate the problems described above, the first artificial-intervertebral-disk insertion jig according to the invention is characterized by comprising an upper jig and a lower jig, as a pair of jigs, which have been fitted to each other so as not to separate from each other in vertical directions and as to be capable of sliding against each other in back-and-forth directions, the upper jig and the lower jig having an upper holding part and a lower holding part which serve to hold an artificial intervertebral disk and have been formed as front parts of the upper jig and lower jig.

The first artificial-intervertebral-disk insertion jig desirably is one in which either of the upper jig and the lower jig has a ridge having a sectional shape with an expanded top and the other has a groove having a sectional shape with an expanded bottom so as to correspond to the ridge and the ridge and the groove have been fitted to each other, whereby the upper jig and the lower jig have been fitted to each other so as not to separate from each other in vertical directions and as to be capable of sliding against each other in back-and-forth directions. Furthermore, the upper holding part of the upper jig and the lower holding part of the lower jig respectively desirably have slots for holding the tips of each pin which protrude respectively from the upper and lower sides of the artificial intervertebral disk. It is also desirable that the front end of the upper holding part of the upper jig and the front end of the lower holding part of the lower jig should have been formed in a semicircular shape or semi-elliptic shape. The upper jig and the lower jig desirably are made of ultrahigh-molecular polyethylene. The upper jig and the lower jig desirably are circular-arc-shaped bent jigs having the same radius of curvature and have been fitted to each other so as to be capable of sliding against each other in the circular-arc directions which are the back-and-forth directions for the jigs. It is further desirable that either of the upper jig and the lower jig should have a stopper which inhibits the jig from sliding forward from a position in which the upper jig overlies the lower jig. Moreover, the artificial-intervertebral-disk insertion jig desirably has a handle attached to the rear end of the upper jig through a shaft.

The second artificial-intervertebral-disk insertion jig according to the invention is characterized by comprising a block having an upper holding part and a lower holding part which serve to hold an artificial intervertebral disk, a pipe attached to a rear part of the block, and a shaft which has been inserted in the pipe in a freely slidable manner and has an artificial-intervertebral-disk support piece attached to the front end of the shaft, the support piece being located in a recessed space formed at the back of the space between the upper holding part and lower holding part of the block.

On the other hand, the jig set of the invention is characterized in that it comprises the first artificial-intervertebral-disk insertion jig described above and an insertion guide jig having a guide hole, and that the insertion guide jig, when the insertion jig is inserted in the guide hole, guides the insertion jig so that the artificial intervertebral disk held by the upper holding part and lower holding part reaches a given position between vertebrae.

The artificial intervertebral disk according to the invention is characterized by comprising: a core material comprising a structure which is either a three-dimensional woven structure or knit structure made of organic fibers arranged along three or more axes or a structure comprising a combination of the woven structure and the knit structure; and plates superposed respectively on the upper and lower sides of the core material, the plates being made of a biodegradable and bioabsorbable polymer containing bioactive bioceramic particles.

In the artificial intervertebral disk of the invention, the plates preferably are as follows from the standpoint of physicochemical properties such as, e.g., high initial strength and an appropriate strength retention period. Namely, the plates each preferably are a forging of a biodegradable and bioabsorbable polymer containing bioactive bioceramic particles. It is also preferred from the standpoint of bone cell penetration that many perforations be formed in the plates and that the rate of openings of the plates be regulated to 15-60%.
Furthermore, it is preferred that the perforations of the plates should be partly or wholly filled with a biodegradable and bioabsorbable material having bone conductivity and/or bone inductivity and excellent bioactivity and undergoing degradation in the living body at a higher rate than the plates, and that the plates each should have a porous covering layer formed on the obverse side thereof or on each of the obverse and reverse sides thereof, the covering layer being made of the biodegradable and bioabsorbable material. The biodegradable and bioabsorbable material preferably is a porous obj ect of a biodegradable and bioabsorbable polymer, the porous object having interconnected pores inside and containing bioceramic particles having bone conductivity and/or any of a cytokine, a drug, and a bone induction factor each having bone inductivity. Alternatively, the biodegradable and bioabsorbable material preferably is one obtained by incorporating bioactive bioceramic particlesor a bone induction factor into collagen. It is also preferred to form fine recesses and protrusions on each side of each plate and to form projections on the obverse side of each plate. Furthermore, it is preferred that biodegradable and bioabsorbable pins be disposed so that they extend through the core material and the plates and the tips of each pin protrude from the plate surfaces, and that the periphery of each plate be sewed to the core material with a yarn.

The first artificial-intervertebral-disk insertion jig of the invention can be used in the following manner. First, an artificial intervertebral disk is held by the upper holding part of the upper jig and the lower holding part of the lower jig, and this insertion jig is inserted between adjacent vertebral bodies either from the back side of the vertebral column or from a lateral side for the purpose of attaining low invasion and avoiding contact with the nerve root. Subsequently, the upper jig is slid backward and drawn out while keeping the lower jig fixed. While the upper side of the artificial intervertebral disk is kept in the state of being lightly pressed against the upper vertebral body, the lower jig is drawn out to cause the artificial intervertebral disk to be sandwiched between the upper and lower vertebral bodies. Thus, the artificial intervertebral disk can be easily inserted between the upper and lower vertebral bodies without fail. Consequently, this artificial-intervertebral-disk insertion jig is excellent in controllability and handleability.
In particular, the artificial-intervertebral-disk insertion jig wherein the upper jig and the lower jig are circular-arc-shaped bent jigs having the same radius of curvature and have been fitted to each other so as to be capable of sliding against each other in the circular-arc directions which are the back-and-forth directions for the jigs is highly excellent in controllability and handleability because this jig can be inserted between vertebrae from the back side of the vertebral column in a circular-arc direction through the space among the excised vertebral arc and intervertebral joint and an artificial intervertebral disk can be inserted in a proper position and proper direction.

The artificial-intervertebral-disk insertion jig
wherein either of the upper jig and the lower jig has a ridge having a sectional shape with an expanded top and the other has a groove having a sectional shape with an expanded bottom so as to correspond to the ridge and the ridge and the groove have been fitted to each other has the following advantage. Since the ridge and the groove are engaged with each other in vertical directions and the ridge smoothly slides in the groove while inhibiting the upper jig and lower jig from separating from each other in vertical directions, it is possible to smoothly slide and easily draw out the upper jig while keeping the lower jig fixed.
In particular, the artificial-intervertebral-disk insertion jig in which the upper jig and the lower jig are made of ultrahigh-molecular polyethylene has a low coefficient of friction and high slip properties and, hence, is effective in facilitating the manipulation in which the insertion jig is inserted between vertebrae from the back side, the manipulation in which the upper jig is slid and drawn out, and the manipulation in which the lower jig is drawn out, leaving the artificial intervertebral disk. In addition, since ultrahigh-molecular polyethylene has high hardness and high strength, this insertion jig is less apt to break.

The artificial-intervertebral-disk insertion jig wherein the upper holding part of the upper jig and the lower holding part of the lower jig respectively have slots for holding the tips of each pin which protrude respectively from the upper and lower sides of an artificial intervertebral disk has the following advantages. Even when an artificial intervertebral disk having pins whose tips protrude from the upper and lower sides of the disk is used, this artificial intervertebral disk can be held by the upper holding part and the lower holding part and insertedbetween adj acent vertebral bodies while keeping the tips of the pins in the state of being held in the slots. Since the pin tips are not caught by the upper and lower vertebral bodies, there is no possibility that the insertion of the artificial intervertebral disk might be prevented. Upon the drawing of the upper jig, the pin tips protruding from the upper side of the artificial intervertebral disk stick into the upper vertebral body to fix the artificial intervertebral disk. Because of this, the artificial intervertebral disk does not suffer positional shifting when the lower jig is drawn out. After the lower jig has been drawn out, the pin tips protruding from the lower side also stick into the lower vertebral body. Consequently, the artificial intervertebral disk is fixed as a stand-alone disk without fail.

The artificial-intervertebral-disk insertion jig wherein the front end of the upper holding part of the upper jig and the front end of the lower holding part of the lower jig have been formed in a semicircular shape or semi-elliptic shape has the following advantages. The resistance which the insertion jig receives during insertion between adjacent vertebral bodies is low, and there is no possibility that even when the front end of the upper or lower holding part hits against a vertebral body, this neither damages the vertebral body nor prevents the insertion because the front end thereof is not angular.

The artificial-intervertebral-disk insertion jig wherein either of the upper jig and the lower jig has a stopper which inhibits the jig from sliding forward from a position in which the upper jig overlies the lower jig has the following advantage. By pressing and pushing the upper jig inward, the insertion jig can be inserted between adjacent vertebral bodies while keeping the upper jig and lower jig in the state of being superposed on each other. Consequently, the upper jig and lower jig can be prevented, during insertion, from suffering forward or backward positional shifting and thereby making the holding of the artificial intervertebral disk by the upper holding part and lower holding part incomplete.

When the artificial-intervertebral-disk insertion jig described above in which either of the upper jig and the lower jig has a stopper is one which has a handle attached to the rear end of the upper jig through a shaft, then this insertion jig has further improved controllability and handleability because the manipulation of inserting an artificial intervertebral disk and the manipulation of drawing out the upper jig can be conducted, with this handle gripped by the operator.

Furthermore, the second artificial-intervertebral-disk insertion jig of the invention can be used in the following manner. An artificial intervertebral disk is held by the upper holding part and lower holding part of the block, and this insertion jig is inserted between cervical vertebral bodies from the front side. The shaft is fixed in a position, and the pipe is drawn out together with the block while supporting the artificial intervertebral disk with the artificial-intervertebral-disk support piece at the shaft front end so as to prevent the artificial intervertebral disk from coming out from the space between the vertebral bodies. Thus, the artificial intervertebral disk can be easily and precisely inserted and disposed between the vertebral bodies. It is also possible to insert and dispose an artificial intervertebral disk between lumbar vertebral bodies from the front side by using this insertion jig in the same manner.

On the other hand, the jig set of the invention can be used in the following manner. The insertion guide jig is disposed at the back of the space between vertebrae. The first artificial-intervertebral-disk insertion jig is inserted from the back side into the guide hole of the insertion guide jig. Thus, the artificial intervertebral disk held by the upper holding part and lower holding part of the insertion jig can be precisely guided and inserted to a given position between the vertebrae.

When the artificial-intervertebral-disk insertion jig or jig set of the invention is used to insert the artificial intervertebral disk of the invention between adjacent vertebral bodies, the artificial intervertebral disk of the invention sufficiently functions as an intervertebral disk because the core material, which comprises a structure which is either a multiaxis three-dimensional woven structure or knit structure made of organic fibers arranged along three or more axes or a structure comprising a combination of the woven structure and the knit structure, has almost the same mechanical strength and flexibility as intervertebral disks of the living body and the deformation properties thereof are highly biomimetic. In addition, since the plates superposed on the core material are plates made of a biodegradable and bioabsorbable polymer containing bioceramic particles, hydrolysis and absorption proceed from the plate surfaces upon contact with a body fluid. With this degradation/absorption, bone tissues grow conductively toward inner parts of the plates due to the bone conductivity of the bioceramic particles. Finally, the plates are replaced by bone tissues and the core material directly bonds to the vertebral bodies. In this case, the plates made of a biodegradable and bioabsorbable polymer have a lower rate of degradation/absorption than spacers comprising a porous object and the degradation/absorption rate thereof is substantially balanced with the rate of growth of bone tissues. Consequently, the plates are gradually destroyed with the degradation/absorption thereof. Simultaneously therewith, bone tissues grow and directly bond to the plates. Thereafter, the plates are gradually degraded and absorbed and, finally, the artificial intervertebral disk comprising the core directly bonds to the vertebral bodies. Thus, the force of bonding and fixing to the vertebral bodies can be secured. In addition, since the plates made of a biodegradable and bioabsorbable polymer are not brittle, the plates can be prevented from generating fine particles even when the artificial intervertebral disk repeatedly undergoes biomimetic deformations under the high sandwiching pressure of the upper and lower vertebral bodies.

The artificial intervertebral disk of the invention wherein the plates each are a forging of a biodegradable and bioabsorbable polymer containing bioactive bioceramic particles has the following advantage. Forging not only compresses the polymer to densify the plates but also orients the polymer molecules, crystals, etc., as will be described later, to improve the strength of the plates. Because of this, even when the plates are repeatedly deformed together with the core material in the living body by the pressure imposed by bones of the living body, much time is required for the plates to be deprived of their mechanical strength and destroyed. By the time when the plates are thus destroyed, bonding between the artificial intervertebral disk and the vertebral bodies is secured due to bone conduction, i.e., the conduction and penetration of living-body bones.

The artificial intervertebral disk wherein the plates have many perforations formed therein brings about the following advantage. Since a body fluid passes through the perforations of the plates and reaches the back sides of the plates to cause degradation/absorption to proceed also on the plate back sides, bone tissues can grow on both sides of each plate to attain direct bonding to the vertebral bodies in an early stage. The artificial intervertebral disk in which the plates have a rate of openings of 15-60% has the following advantage. The plates have a strength which enables the plates to withstand the sandwiching pressure of the upper and lower vertebral bodies, and the plates as a whole have a moderate rate of degradation/absorption. Because of this, the plates can be completely replaced by bone tissues to attain tenacious bonding to the vertebral bodies.

The artificial intervertebral disk wherein the perforations of the plates are partly or wholly filled with a biodegradable and bioabsorbable material having bone conductivity and/or bone inductivity and undergoing degradation in the living body at a higher rate than the plates has the following advantages. The biodegradable and bioabsorbable material with which the perforations are filled is degraded more rapidly than the plates, and due to the bone conductivity and/or bone inductivity thereof, bone tissues rapidly grow conductively and/or inductively. The biodegradable and bioabsorbable material in the perforations is thus replaced by bone tissues in an early stage. On the other hand, the degradation of the plates proceeds more slowly than that of the biodegradable and bioabsorbable material, and the plates are present at the interface between each vertebral body and the artificial intervertebral disk itself and retain sufficient strength until the biodegradable and bioabsorbable material in the perforations is replaced by bone tissues to some degree, whereby securing bonding to the vertebral bodies. Thus, the plates play an important role for completing a stand-alone artificial intervertebral disk. Thereafter, the plates are wholly replaced by bone tissues.

The artificial intervertebral disk wherein the plates each have a covering layer made of the same biodegradable and bioabsorbable material on the front side thereof or on each of the obverse and reverse sides thereof has an advantage that bone tissues almost evenly grow on the plate surfaces in an early stage.

The artificial intervertebral disk of the invention wherein the plates each have fine recesses and protrusions formed on each side thereof has the following advantages. The protrusions of the recesses and protrusions on the front side of each plate bite into the vertebral body to prevent the artificial intervertebral disk from suffering positional shifting/falling off. Furthermore, the recesses and protrusions on the front side are effective in bonding because they considerably increase the area of contact with the vertebral body. On the other hand, the protrusions of the recesses and protrusions on the back side of each plate bite into the core material and thereby prevent the plate and the core material from suffering relative positional shifting. The artificial intervertebral disk wherein the plates each have projections formed on the front side thereof also has the advantage of being capable of preventing positional shifting/falling off because the projections stick into the vertebral bodies.

Furthermore, the artificial intervertebral disk which has at least one biodegradable and bioabsorbable pin extending through the core material and the plates so that the tips of the pin protrude from the plate surfaces has advantages that relative positional shifting and separation between each plate and the core material can be prevented and that the protruding tips of the pin bite into the vertebral bodies, whereby the artificial intervertebral disk can be prevented from suffering positional shifting/falling off. Moreover, the artificial intervertebral disk wherein the periphery of each plate has been sewed to the core material with a yarn has an advantage that relative positional shifting and separation between each plate and the core material can be prevented. Consequently, there is no need of conducting the intervertebral fixing with an auxiliary metallic device which is necessary after the disposition of various fusion cages.

### BRIEF DESCRIPTION OF THE DRAWINGS

Fig. 1 is a slant view illustrating one embodiment of the first artificial-intervertebral-disk insertion jig according to the invention.
Fig. 2 is a plan view of the insertion jig.
Fig. 3 is a plan view illustrating the insertion jig in which the upper jig has been slid.
Fig. 4 (a) is a sectional view taken on the line A-A of Fig. 2 and (b) is a sectional view taken on the line B-B of Fig. 2.
Fig. 5 (a) is a bottom view of the upper jig of the insertion jig and (b) is a plan view of the lower jig of the insertion jig.
Fig. 6 is an exploded partial side view of the rear end part of the insertion jig.
Fig. 7 is a slant view illustrating one embodiment of the artificial intervertebral disk according to the invention.
Fig. 8 is a slant view of the insertion jig which holds the artificial intervertebral disk.
Fig. 9 is a sectional view taken on the line C-C of Fig. 8.
Fig. 10 is a view illustrating a method of using the insertion jig; the view shows the stage at which the insertion jig holding the artificial intervertebral disk has been inserted between adjacent intervertebral bodies.
Fig. 11 is a view illustrating the method of using the insertion jig; the view shows the stage at which the upper jig of the insertion jig holding the artificial intervertebral disk has been drawn out.
Fig. 12 is a view illustrating the method of using the insertion jig; the view shows the stage at which the lower jig has been drawn out and the artificial intervertebral disk has been sandwichedbetween the upper and lower vertebral bodies.
Fig. 13 is a plan view showing the insertion positions of a pair of artificial intervertebral disks, as right and left disks, which each are the artificial intervertebral disk according to the invention.
Fig. 14 is a plan view showing the insertion positions of a pair of artificial intervertebral disks, as right and left disks, which each are the artificial intervertebral disk according to the invention and of an intermediate artificial intervertebral disk according to another embodiment of the invention.
Fig. 15 is a slant view illustrating another embodiment of the first artificial-intervertebral-disk insertion jig according to the invention.
Fig. 16 (a) is a bottom view of the upper jig of the insertion jig and (b) is a plan view of the lower jig of the insertion jig.
Fig. 17 is a slant view illustrating still another embodiment of the first artificial-intervertebral-disk insertion jig according to the invention.
Fig. 18 is a plan view illustrating one embodiment of the jig set according to the invention.
Fig. 19 is a view illustrating a method of using the jig set.
Fig. 20 (a) is a plan view illustrating one embodiment of the second artificial-intervertebral-disk insertion jig according to the invention and (b) is a sectional view thereof taken on the line D-D.
Fig. 21 is views illustrating a method of using the insertion jig; (a) shows the stage at which the block holding an artificial intervertebral disk has been inserted between cervical vertebrae, (b) shows the stage at which a spacer has been removed from the handle, and (c) shows the stage at which the block has been drawn out of the space between the vertebrae while keeping the artificial intervertebral disk supported by the artificial-intervertebral-disk support piece.
Fig. 22 is a slant view illustrating another embodiment of the artificial intervertebral diskaccording to the invention.
Fig. 23 is a slant view illustrating still another embodiment of the artificial intervertebral disk according to the invention.
Fig. 24 is an enlarged sectional view illustrating a further embodiment of the artificial intervertebral disk according to the invention.
Fig. 25 is an enlarged sectional view illustrating still a further embodiment of the artificial intervertebral disk according to the invention.
Fig. 26 is,a slant view illustrating still a further embodiment of the artificial intervertebral disk according to the invention.
Fig. 27 is a slant view illustrating still a further embodiment of the artificial intervertebral disk according to the invention.

### DESCRIPTION OF REFERENCE NUMERALS AND SIGNS

- 1: upper jig
- 1a: upper holding piece
- 1b: ridge
- 1d: slot for holding pin tip
- 1f: stopper
- 2: lower jig
- 2a: lower holding piece
- 2b: groove
- 2d: slot for holding pin tip
- 3, 30, 31, 32, 33, 34, 35, 36: artificial intervertebral disk
- 3a: core material comprising fibrous structure
- 3b: plate
- 3c: pin
- 3d: pin tip
- 3e: large perforation
- 3f: small perforation
- 3g: biodegradable and bioabsorbable material undergoing rapid degradation
- 3h: covering layer made of biodegradable and bioabsorbable material undergoing rapid degradation
- 4: shaft
- 5: handle
- 6: insertion guide jig
- 6a: guide hole
- 10, 11: artificial-intervertebral-disk insertion jig
- 11a: block
- 11b: pipe
- 11c: shaft
- 11d: artificial-intervertebral-disk support piece
- 11i: upper holding part
- 11j: lower holding part
- 11m: recessed space
- 20: vertebral (especially lumbar vertebral) body
- 21: cervical vertebral body

### BEST MODE FOR CARRYING OUT THE INVENTION

Embodiments of the invention will be explained below in detail by reference to the drawings.

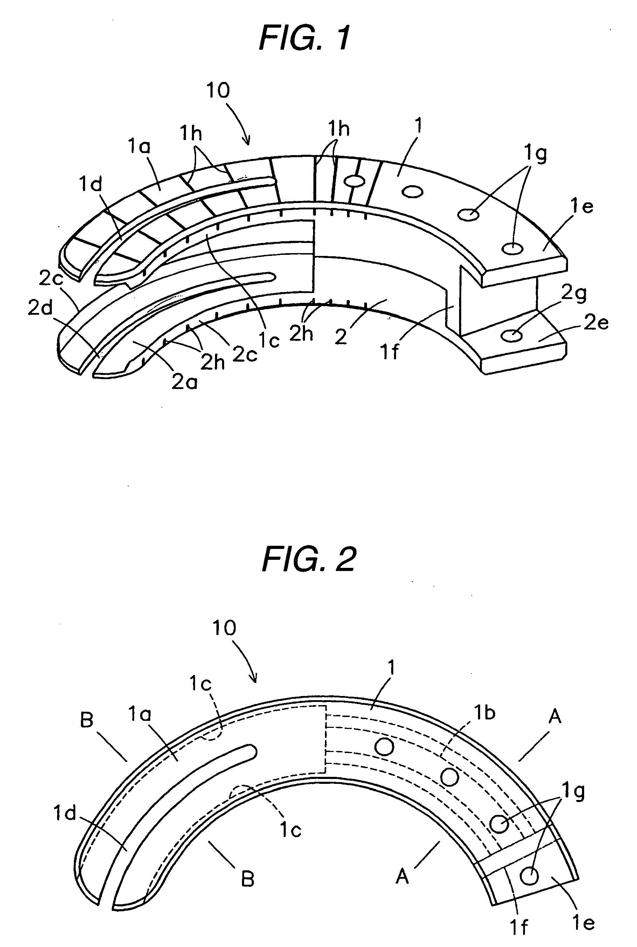

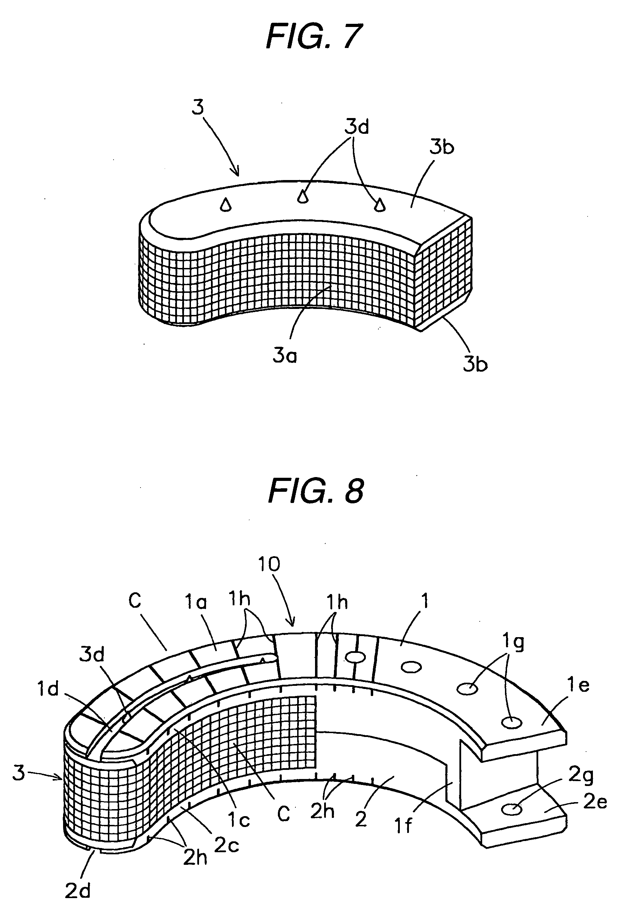

The first artificial-intervertebral-disk insertion jig 10 shown in Figs. 1 to 6 comprises a pair of jigs, i.e., an upper jig 1 and a lower jig 2, which are circular-arc-shaped bent jigs having the same radius of curvature. The upper jig 1 and the lower jig 2 have, formed as front parts thereof, an upper holding part 1a and a lower holding part 2a which each are in the form of a tongue and serve to hold a circular-arc-shaped artificial intervertebral disk 3 shown in Fig. 7.

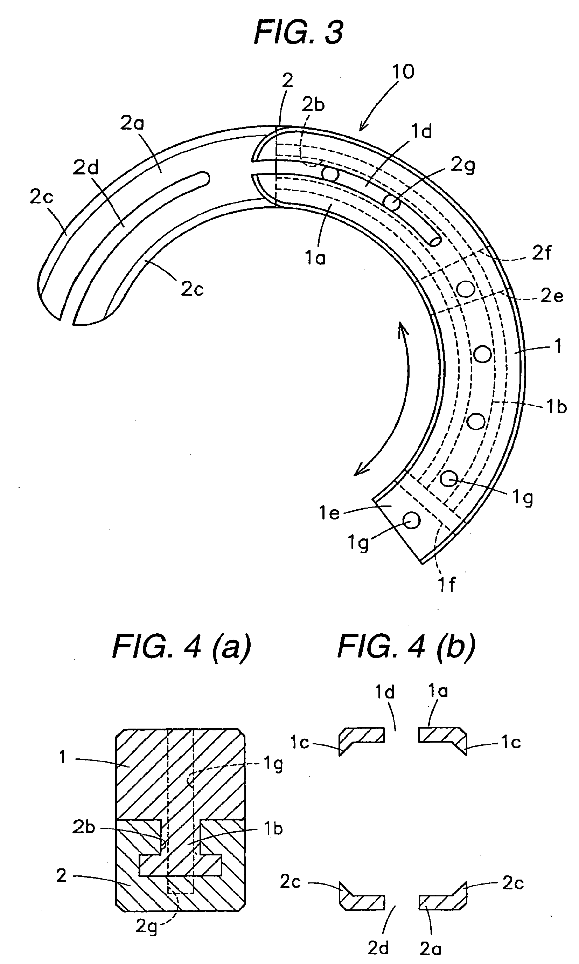

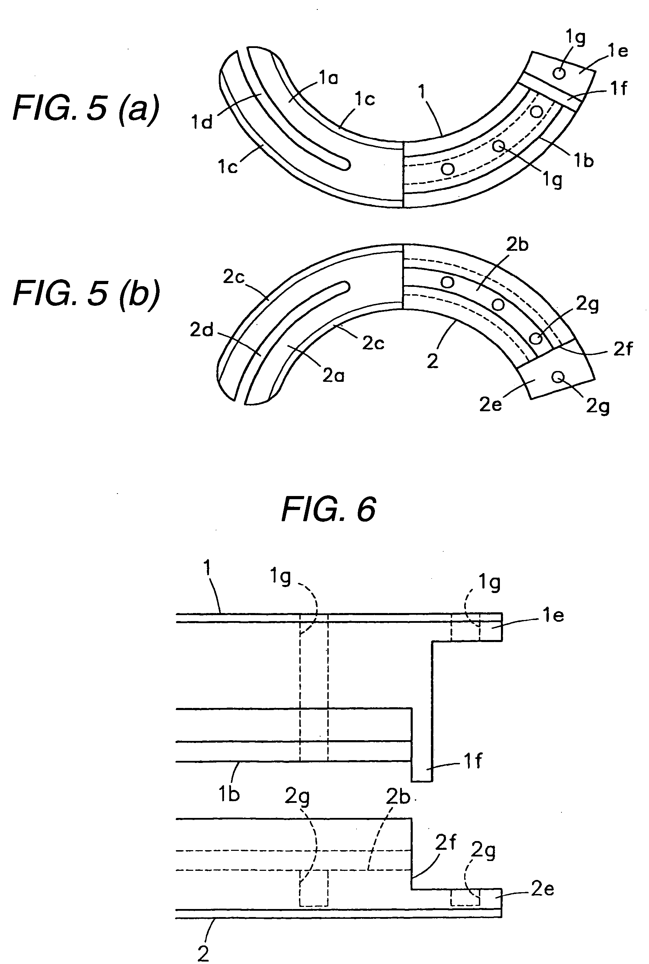

As shown in Fig. 4 (a) and Fig. 5 (a), the rear part (the thick part on the rear side of the upper holding part 1a) of the upper jig 1 has, on its lower side, a ridge 1b which has a cross section of an inverted-T shape and is curved along the center line (not shown in the figures) of the upper jig 1. The rear part (the thick part on the rear side of the lower holding part 2a) of the lower jig 2 has, on its upper side, a groove 2b which has a cross section of an inverted-T shape and is curved along the center line of the lower jig 2, as shown in Fig. 4 (a) and Fig. 5 (b), so as to correspond to the ridge 1b. The ridge 1b and the groove 2b have been fitted to each other as shown in Fig. 4 (a), whereby the upper jig 1 and the lower jig 2 have been fitted to each other so as not to separate from each other in vertical directions and as to be slidable against each other in circular-arc directions (back-and-forth directions) as shown in Fig. 3.

The sectional shape of the ridge 1b and groove 2b is not limited to the inverted-T shape, and the ridge 1b and groove 2b may have any sectional shape which enables the ridge 1b and groove 2b to engage with each other and prevents these from separating from each other in vertical directions. Namely, the ridge may have any sectional shape with an expanded top, while the groove may have the corresponding sectional shape with an expanded bottom. Although the upper jig 1 has a ridge 1b on its lower side and the lower jig 2 has a groove 2b on its upper side in this embodiments, a reverse constitution is possible in which the upper jig 1 has a groove 2b formed on its lower side and the lower jig 2 has a ridge 1b formed on its upper side. Furthermore, although the ridge 1b in this embodiment has been formed in a continuous circular-arc shape, it may be discontinuous.

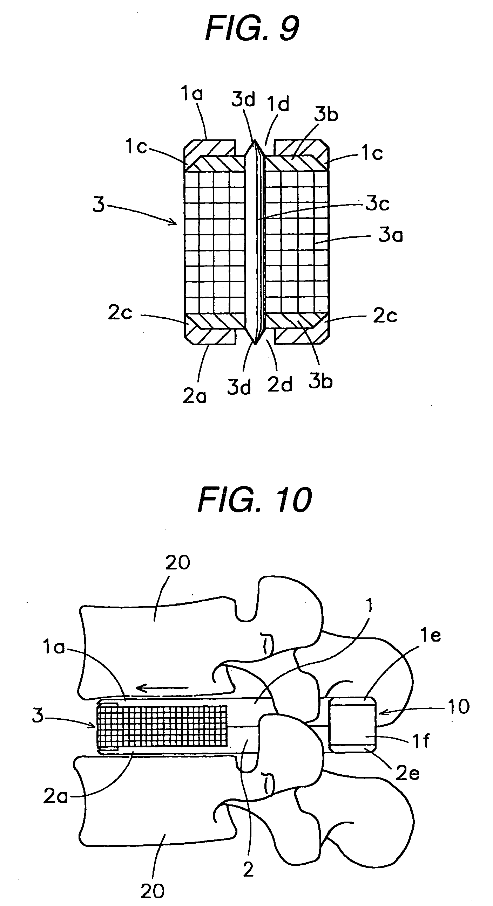

As shown in Fig. 1, Fig. 4 (b), and Fig. 5 (a) and (b), the upper holding part 1a has, at both side edges thereof, flanges 1c and 1c having a right-triangle sectional shape projecting downward. Likewise, the lower holding part 2a has, at both side edges thereof, flanges 2c and 2c having a right-triangle sectional shape projecting upward. The upper holding part 1a and the lower holding part 2a respectively have slits 1d and 2d for holding the tips 3d of each pin which protrude respectively from the upper and lower sides of an artificial intervertebral disk 3 (see Fig. 7). These slits 1d and 2d have been formed so as to extend from the front ends of the upper and lower holding parts 1a and 2a and be curved along the respective center lines. Consequently, when an artificial intervertebral disk 3 is sandwiched between the upper holding part 1a and the lower holding part 2a, not only the artificial intervertebral disk 3 is prevented from suffering rightward or leftward positional shifting due to the flanges 1c, 1c, 2c, and 2c, but also the pin tips 3d protruding from the upper and lower sides of the artificial intervertebral disk 3 are held in the slits 1d and 2d and kept in the state of not protruding from the upper and lower holding parts 1a and 2a, as shown in Fig. 9. Incidentally, the slots for holding the pin tips 3d are not limited to slits 1d and 2d as in this embodiments, and may be grooves respectively formed on the lower side of the upper holding part 1a and the upper side of the lower holding part 2a.

Furthermore, the front ends of the upper holding part 1a and lower holding part 2a have been formed in a semicircular shape which is not angular. Because of this, the resistance which this artificial-intervertebral-disk insertion jig 10 receives during insertion between vertebrae is reduced. The semicircular shape is intended also to produce the effect that even when the front end of the upper or lower holding part 1a or 2a hits against a vertebral body, this neither damages the vertebral body nor prevents the insertion. The shape of each front end is not limited to semicircular shapes, and may be any round shape which is not angular, such as, e.g., a semi-elliptic shape.

As shown in Figs. 1 and 6, the upper jig 1 and the lower jig 2 respectively have a top plate 1e and a bottom plate 2e which extend from the rear ends of the jigs 1 and 2. The top plate 1e and bottom plate 2e respectively have holes 1g and 2g for inserting the front end of a hook rod thereinto. Also in the thick rear part of the upper jig 1, holes 1g for inserting the front end of a hook rod thereinto have been formed at a given interval. The thick rear part of the lower jig 2 also has holes 2g for inserting the front end of a hook rod thereinto, these holes 2g corresponding to the holes 1g. Consequently, the upper jig 1 or lower jig 2 can be easily drawn out with a hook rod, with the front end of the hook rod inserted in any of these holes 1g and 2g.

The upper jig 1 further has a platy stopper 1f formed at the rear end thereof so as to project downward as shown in Figs. 1 and 6. When the upper jig 1 is in a position in which it overlies the lower jig 2 as shown in Figs. 1 and 2, the stopper 1f is in contact with a rear end surface 2f of the lower jig 2 (see Fig. 6) and inhibits the upper jig 1 from sliding forward from this position. When such a stopper 1f has been formed, merely pushing the upper jig 1 inward enables the upper jig 1 and the lower jig 2 to be inserted between adjacent vertebral bodies while keeping the upper jig 1 and the lower jig 2 in the superposed state. It is therefore possible to prevent the upper jig 1 and lower jig 2 from suffering forward or backward positional shifting during insertion and thus making the holding of an artificial intervertebral disk 3 by the upper and lower holding parts 1a and 2a incomplete.
In addition, when the lower jig 2 is drawn out, the upper jig 1 also is drawn out together with the lower jig 2. Consequently, the manipulation for regulating the insertion position of the artificial intervertebral disk 3 can be facilitated.

In this embodiment, the stopper 1f has been formed at the rear end of the upper jig 1. However, a reverse constitution is possible in which a stopper projecting upward is formed in a central part of the lower jig 2 (i.e., at the root of the lower holding part 2a) so that this stopper is in contact with an end surface of a central part of the upper jig 1 when the upper jig 1 overlies the lower jig 2.

As shown in Figs. 1 and 8, the upper jig 1 has incised lines 1h which are slightly recessed dimension lines. Likewise, the lower jig 2 also has incised lines 2h. These incised lines 1h and 2h have been formed at an interval of 1-5 mm in the upper jig 1 and lower jig 2. The incised lines 1h and 2h are intended to enable the operator to know the distance in mm over which the upper or lower jig 1 or 2 has moved toward the space between vertebrae or come out from the intervertebral space when this artificial-intervertebral-disk insertion jig 10 is inserted between the vertebrae and drawn out therefrom. In this embodiment, the pitch of the incised lines 1h and 2h in the upper holding part 1a and lower holding part 2a is two times the pitch of the incised lines 1h and 2h in the main body parts of the upper jig 1 and lower jig 2. However, the pitches may, of course, be the same.

The material of the insertion jig 10 may be either a metal or a synthetic resin as long as it has high strength. However, ultrahigh-molecular polyethylene is especially preferred of such materials. This insertion jig made of ultrahigh-molecular polyethylene has high hardness and high strength and is hence less apt to break. In addition, there is an advantage that since this insertion jig has a low coefficient of friction and high slip properties, it is effective in facilitating the manipulation in which the insertion jig 10 is inserted between vertebrae from the back side, the manipulation in which the upper jig 1 is slid and drawn out, and the manipulation in which the lower jig 2 is drawn out, leaving the artificial intervertebral disk 3.

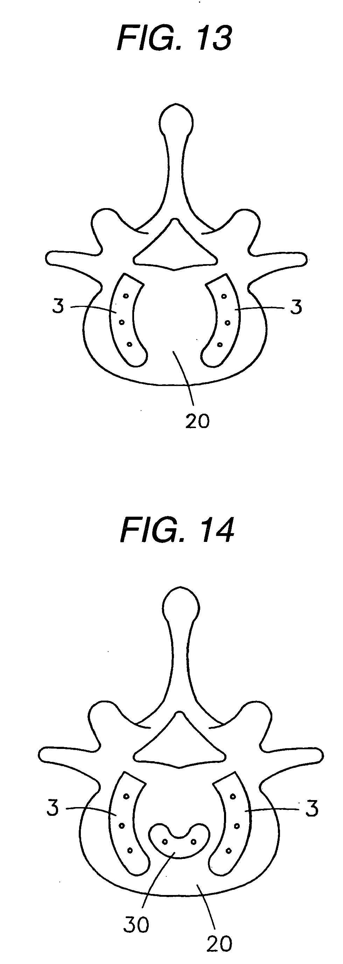

As shown in Figs. 7 and 9, the artificial intervertebral disk 3 of the invention to be inserted between adjacent vertebrae with the insertion jig 10 described above is a circular-arc-shaped artificial intervertebral disk obtained by superposing plates 3b and 3b comprising a biodegradable and bioabsorbable polymer (e. g. , poly (lactic acid) or the like) containing 25-60% by mass bioactive bioceramic particles (e.g., unsintered hydroxyapatite or the like) respectively on the upper and lower sides of a core material 3a comprising a structure which is either a multiaxis three-dimensional woven structure or knit structure made of bioinert organic fibers (e.g., coated fibers obtained by coating core fibers of ultrahigh-molecular polyethylene with a film of linear low-density polyethylene) arranged along three or more axes or a structure comprising a combination of the woven structure and the knit structure and disposing pins 3c for fixing made of the same biodegradable and bioabsorbable polymer so that the pins 3c vertically extend through the core material 3a and the plates 3b and 3b and the tips 3d of the pins protrude from the upper and lower surfaces. As shown in Fig. 13, a pair of such artificial intervertebral disks 3 and 3, which are one curved clockwise and one curved counterclockwise, are inserted between vertebrae in symmetrical positions with respect to right-and-left symmetry. These artificial intervertebral disks 3 and 3 undergo biomimetic deformations similar to those of intervertebral disks of the living body. Specific dimensions of each artificial intervertebral disk 3 are as follows: the radius of curvature (radius of curvature of the center line) is about 15-40 mm, the length (length of the center line) is about 15-35 mm, the width is about 7-15 mm, and the height is about 7-14 mm. The artificial intervertebral disk of the invention will be explained later in detail.

The artificial-intervertebral-disk insertion jig 10 of the invention has been designed to have dimensions suitable for the holding of the artificial intervertebral disk 3 and the insertion thereof between vertebrae. Specifically, the radius of curvature (radius of curvature of the center line) of each of the upper and lower jigs 1 and 2 is about 15-40 mm, the width of each of the upper and lower jigs 1 and 2 is about 7-12 mm, the length (length of the center line) of each of the upper and lower holding parts 1a and 2a is about 15-35 mm, the overall length (overall length of the center line) of each of the upper and lower jigs 1 and 2 is about 50-70 mm, and the total height of the upper and lower jigs 1 and 2 is about 8-16 mm. Incidentally, these dimension values are mere examples, and it is a matter of course that the dimensions can be suitably changed according to the dimensions and shape of the artificial intervertebral disk 3.

Next, a method of using the artificial-intervertebral-disk insertion jig 10 described above will be explained by reference to Figs. 8 to 13.

First, as shown in Figs. 8 and 9, an artificial intervertebral disk 3 is sandwiched between and held by the upper holding part 1a and the lower holding part 2a so that those tips 3d of the pins 3c which protrude from the upper and lower sides of the artificial intervertebral disk 3 are kept being held in the slits 1d and 2d. This manipulation can be easily conducted by sliding the upper jig 1 backward, placing the artificial intervertebral disk 3 on the lower holding part 2a, and sliding the upper jig 1 forward until the stopper 1f comes into contact with the rear end surface 2f of the lower jig 2 to thereby superposed the upper jig 1 on the lower jig 2, as shown in Fig. 3.

After completion of the holding of the artificial intervertebral disk 3, the insertion jig 10 is inserted between adjacent vertebral (especially lumbar vertebral) bodies 20 and 20 from the back side of the vertebral column as shown in Fig. 10, and positioning is conducted so that the artificial intervertebral disk 3 is inserted in a proper position. This insertion manipulation can be easily conducted, for example, by lightly tapping the stopper 1f formed at the rear end of the upper jig 1 with a hammer or the like to push the insertion jig 10 inward while keeping the upper jig 1 and the lower jig 2 in the superposed state. The positioning of the artificial intervertebral disk 3 maybe conducted, for example, by repeating a manipulation in which the front end of a hook rod is inserted into the hole 2g formed in the bottom plate 2e extending from the rear end of the lower jig 2 to slightly draw out the insertion jig 10 while keeping the upper jig 1 and lower jig 2 in the superposed state or the upper jig 1 is slightly pushed inward with a hammer or the like in the manner described above. If possible, the insertion jig 10 may be pushed inward or drawn out by hand.

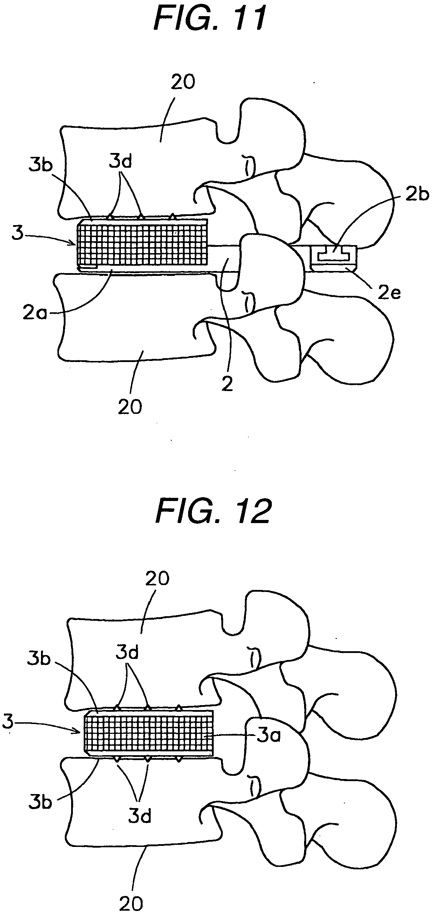

After completion of the manipulation for inserting the insertion jig 10, the front end of a hook rod is inserted into the hole 1g formed in the top plate 1e extending from the rear end of the upper jig 1 to draw out the upper jig while keeping the lower jig 2 fixed, as shown in Fig. 11. As a result of this drawing of the upper jig 1, the upper vertebral body 20 comes into the state of being lightly pressed against the upper plate of the artificial intervertebral disk 3 and the upper tips 3d of the pins stick into the lower side of that vertebral body 20 to fix the artificial intervertebral disk 3.

After completion of the drawing of the upper jig 1, the front end of the hook rod is subsequently inserted into a hole 2g of the lower jig 2 to draw out the lower jig 2 as shown in Fig. 12. In this manipulation, since the artificial intervertebral disk 3 has been fixed to the upper vertebral body 20 with the upper tips 3d of the pins, it is prevented from being drawn out together with the lower jig 1. As a result of this drawing of the lower jig 1, the artificial intervertebral disk 3 is sandwiched between the upper and lower vertebral bodies 20 and 20 and the lower tips 3d of the pins also stick into the lower vertebral body 20. Consequently, the artificial intervertebral disk 3 is completely fixed as a stand-alone disk. Incidentally, even in the case of an artificial intervertebral disk having no pins, this artificial intervertebral disk is prevented from being drawn out together with the lower jig 1 when the lower jig is drawn out, because the artificial intervertebral disk is in press contact with the upper vertebral body 20.
It is, however, desirable that the lower jig 1 in this case be drawn out while holding the artificial intervertebral disk with, e.g., a holding rod applied from the back side, in order to prevent positional shifting of the artificial intervertebral disk without fail.

The insertion of one artificial intervertebral disk 3 is completed in the manner described above. Thereafter, an insertion jig which has the same structure as the insertion jig 10 and is curved in the opposite direction is used to repeat the same manipulations as described above, whereby the other artificial intervertebral disk 3 is inserted into such a position that this artificial intervertebral disk 3 and that artificial intervertebral disk 3 are symmetrically arranged with respect to right-and-left symmetry as shown in Fig. 13.
It is also possible to use the following method. The insertion jig 10 which has been used for inserting one artificial intervertebral disk 3 is used to hold the other artificial intervertebral disk 3, and this insertion jig 10 is turned over so as to be upside-down and then inserted between the vertebrae from the back side. The upper jig 1, which underlies the lower jig 2, is drawn out and the lower jig 2, which is located on the upper side, is then drawn out, whereby the other artificial intervertebral disk 3 is inserted into such a position that this artificial intervertebral disk 3 and that artificial intervertebral disk 3 are symmetrically arranged with respect to right-and-left symmetry as shown in Fig. 13.

In some cases, a comma-shaped artificial intervertebral disk 30 (having the same structure as the artificial intervertebral disks 3) may be inserted between the left and right artificial intervertebral disks 3 and 3 as shown in Fig. 14. It is a matter of course that this insertion of the comma-shaped artificial intervertebral disk 30 can be easily conducted without fail in the same manner as described above by using an insertion jig obtained by modifying the upper holding part 1a and lower holding part 2a of the insertion jig 10 so as to have a shape suitable for holding the comma-shaped artificial intervertebral disk 30.

As described above, the artificial-intervertebral-disk insertion jig 10 of the invention is excellent in controllability and handleability. With this insertion jig 10, a flexible artificial intervertebral disk 3 employing a fibrous structure as a core material can be easily inserted without fail between adjacent vertebral (especially lumber vertebral) bodies 20 and 20 from the back side.

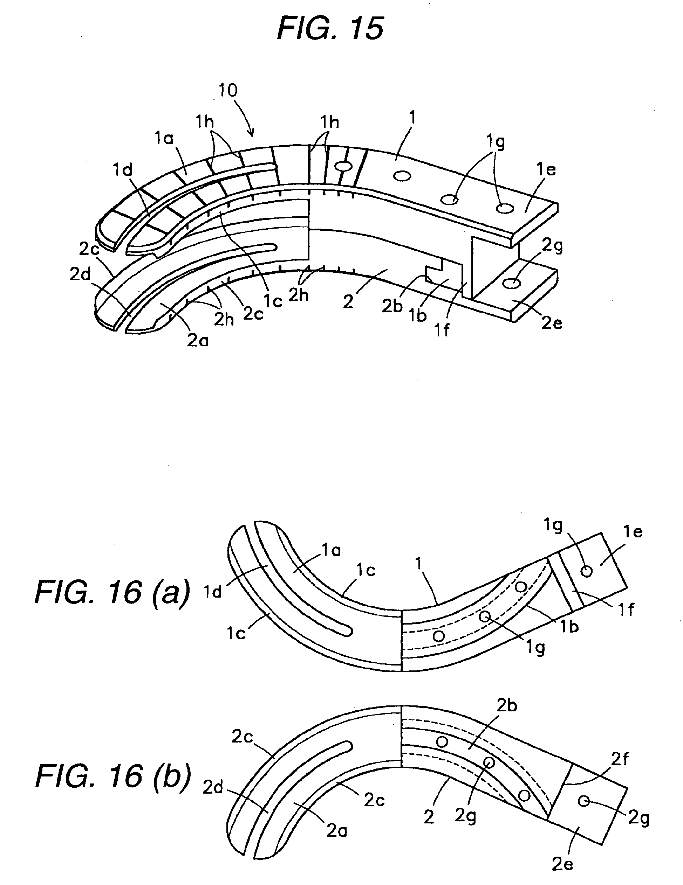

The artificial-intervertebral-disk insertion jig 10 shown as an embodiment in Figs. 15 and 16 comprises an upper jig 1 and a lower jig 2 which are not curved at a constant curvature throughout. That part of the upper jig 1 which is close to the rear end thereof and that part of the lower jig 2 which is close to the rear end thereof extend linearly. Namely, these jigs 1 and 2 have a shape comprising a combination of a circular-arc part and a linear part. It is, however, noted that the ridge 1b formed on the lower side of the upper jig 1 and the groove 2b formed on the upper side of the lower jig 2 are curved at a constant curvature throughout, as shown in Fig. 16 (a) and (b), in order to make the upper jig 1 and the lower jig 2 slidable against each other. The other constitutions are the same as those of the insertion jig 10 described above as the embodiment shown in Figs. 1 to 6. Because of this, like members are designated by like numerals in Figs. 15 and 16, and an explanation is omitted. This insertion jig,
in which those parts of the upper jig 1 and lower jig 2 which are close to the rear ends thereof extend linearly, has an advantage that when the upper jig 1 and lower jig 2 are successively drawn out from the space between vertebrae, the rear ends of the upper jig 1 and lower jig 2 are less apt to hit against a vertebral body (bone) or a nearby biological tissue.

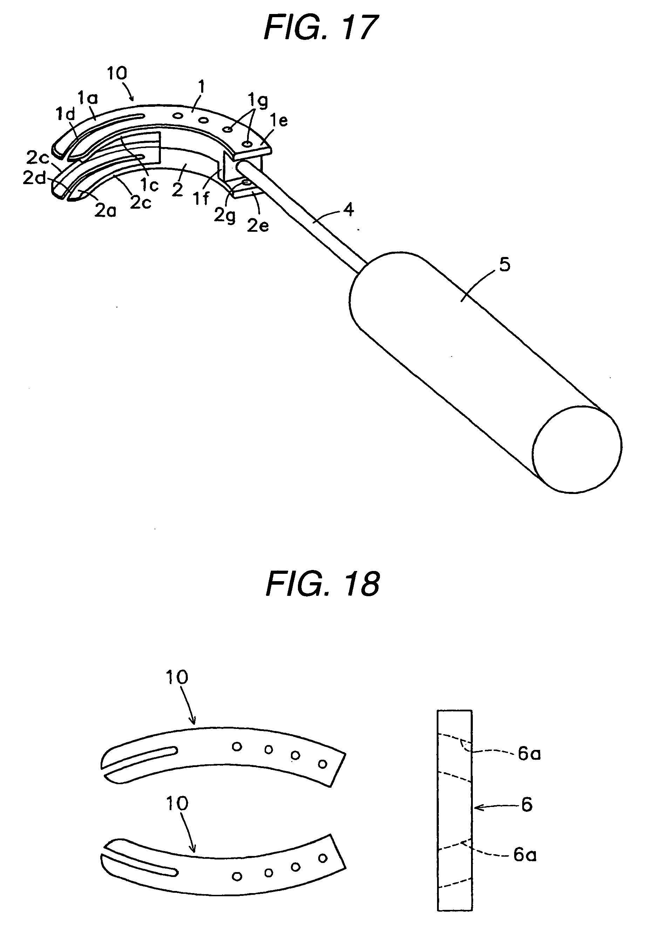

The artificial-intervertebral-disk insertion jig shown as an embodiment in Fig. 17 has a constitution obtained by forming a screw hole in the stopper 1f formed at the rear end of the upper jig 1 of the artificial-intervertebral-disk insertion jig 10 shown as an embodiment in Figs. 1 to 6, screwing the front-end male screw part of a shaft 4 into the screw hole to attach the shaft 4, and disposing a handle 5 through this shaft 4. The constitution of the insertion jig 10 is as described above. Consequently, like members are designated by like numerals in Fig. 17, and an explanation is omitted.

The artificial-intervertebral-disk insertion jig 10 described above has an advantage that it has further improved controllability and handleability because the manipulation for insertion between vertebrae and the manipulation for drawing out the upper jig 1 can be conducted, with the handle 5 gripped by the operator. Incidentally, the artificial-intervertebral-disk insertion jig 10 shown in Figs. 1 to 6 and the artificial-intervertebral-disk jig 10 shown in Fig. 17 not only are used for inserting an artificial intervertebral disk 3 between vertebrae from the back side of the vertebral column (especially lumbar vertebral column) as described above, but also can be made usable for insertion from a lateral side of the vertebral column by making some modifications and contrivances.

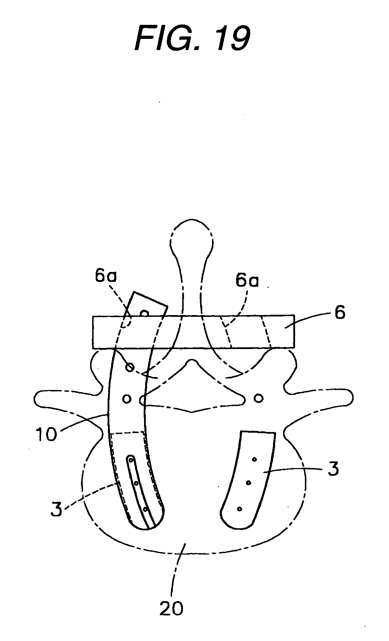

The jig set of the invention shown in Fig. 18 comprises an artificial-intervertebral-disk insertion jig 10 curved leftward, an artificial-intervertebral-disk insertion jig 10 curved rightward, and an insertion guide jig 6. These artificial-intervertebral-disk insertion jigs 10 and 10 have the same constitution as the artificial-intervertebral-disk insertion jig 10 shown as an embodiment in Figs. 1 to 6, except that they have a slightly larger radius of curvature. On the other hand, the insertion guide jig 6 comprises a rectangular-parallelopiped block made of ultrahigh-molecular polyethylene like the insertion jigs 10 and 1,0, and has a pair of guide holes 6a and 6a in which the insertion jigs 10 and 10 are to be inserted. As shown in Fig. 19, the direction of each guide hole 6a is determined, while taking account of the radius of curvature of the insertion jig 10, so that when this insertion guide jig 6 is disposed at the back of the space between vertebrae and each insertion jig 10 is inserted from the back side into the guide hole 6a, then the insertion jig 10 is guided by the guide hole 6a and the artificial intervertebral disk 3 held by the upper and lower holding parts constituting front end parts of the insertion jig 10 reaches a given proper position between the vertebrae.

When the jig set of the invention having such constitution is used, an artificial intervertebral disk 3 held by the upper and lower holding parts constituting front end parts of the insertion jig 10 can be precisely guided to a given position between vertebrae by merely disposing the insertion guide jig 6 at the back of the space between the vertebrae and inserting the insertion jig 10 into the guide hole 6a from the back side. There is hence an advantage that a manipulation for positioning the artificial intervertebral disk 3 is unnecessary and controllability and handleability are greatly improved.

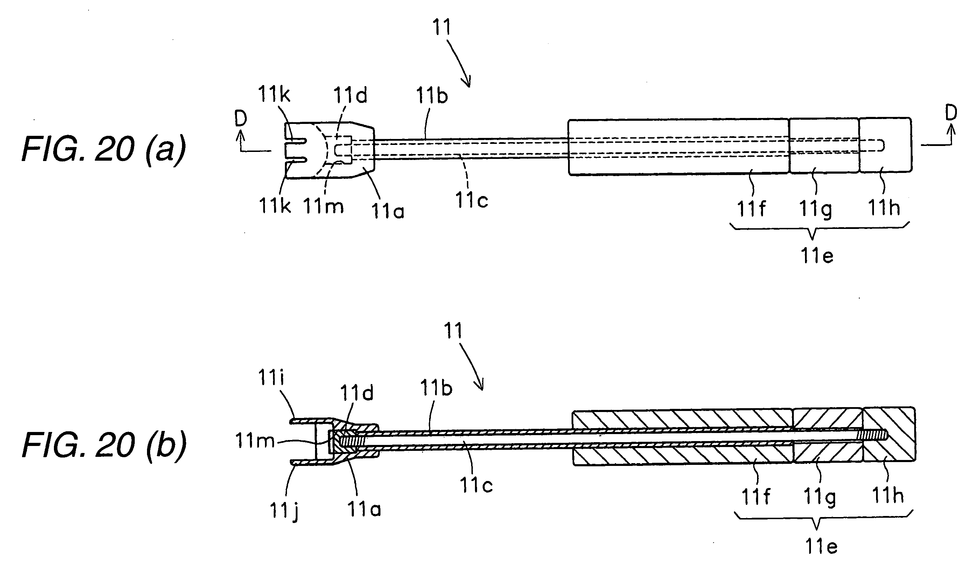

Fig. 20 shows an embodiment of the second artificial-intervertebral-disk insertion jig according to the invention. This insertion jig 11 is constituted of: a block 11a constituting a front end part; a metallic pipe 11b attached to a rear part of the block 11a; a metallic shaft 11c inserted in the pipe 11b in a freely slidable manner; an artificial-intervertebral-disk support piece 11d attached to the front end of the shaft 11c; and a handle 11e. This handle 11e has been divided into a handle main body 11f, a spacer part 11g as an intermediate part, and a falling preventive part 11h constituting a rear end part.

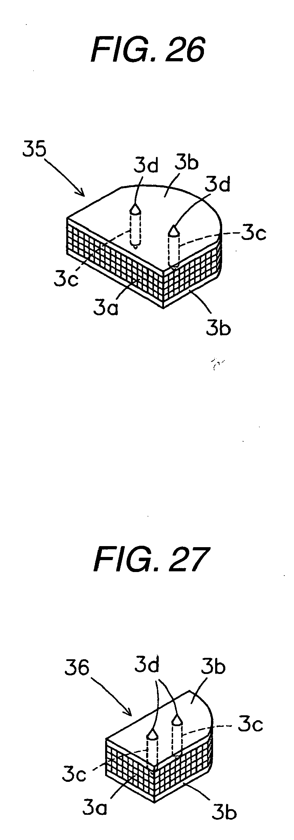

The block 11a constituting a front end part and the artificial-intervertebral-disk support piece 11d are ones made of a harmless synthetic resin or metal, preferably ultrahigh-molecular polyethylene. This block 11a has an upper holding part 11i and a lower holding part 11j which each are in the form of a tongue and which serve to hold an artificial intervertebral disk 35 of a shape square at the front and rounded at the rear (i.e., a shape comprising a combination of a rectangular part as a front half and a semi-circular part as a rear half) such as that shown in Fig. 26. The upper and lower holding parts 11i and 11j each have two parallel slits 11k for holding the pin tips 3d protruding from the upper and lower sides of the artificial intervertebral disk 35.

The front-end male screw part of the shaft 11c has been screwed into a screw hole formed on the rear side of the artificial-intervertebral-disk support piece 11d and this shaft 11c has been fixed with a lock pin (not shown in the figure) to prevent loosening. Thus, the support piece 11d has been attached to the front end of the shaft 11c and is located in a recessed space 11m formed at the back of the space between the upper holding part 11i and lower holding part 11j of the block 11a. The front-side surface of this artificial-intervertebral-disk support piece 11d has been formed as a recessed curved surface so as to conform to the protruding curved rear-side surface of the artificial intervertebral disk 35.

The rear half part of the pipe 11b attached to a rear part of the block 11a constituting a front end part has been inserted in and fixed to the central hole of the handle main body 11f. The shaft 11c, which has been inserted in this pipe 11b in a freely slidable manner, extends through the intermediate spacer part 11g of the handle 11e in a free inserted state, and the rear-end male screw part of the shaft 11c has been screwed into the falling preventive part 11h constituting a rear end part.

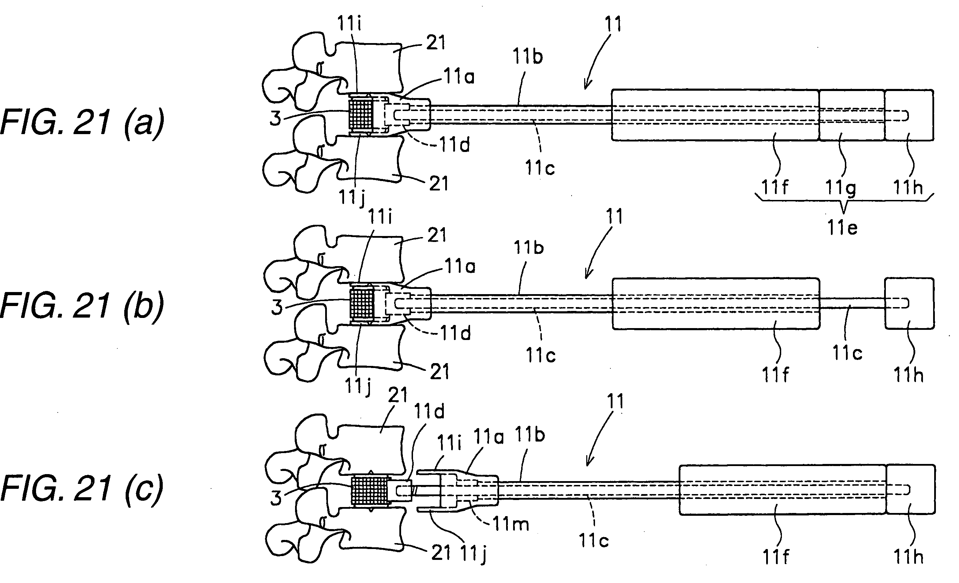

When the artificial-intervertebral-disk insertion jig 11 having the constitution described above is used, an artificial intervertebral disk can be easily inserted between cervical vertebral bodies in the following manner without fail. First, as shown in Fig. 21 (a), the artificial intervertebral disk 35 for cervical vertebrae shown in Fig. 26 is held with the upper holding part 11i and lower holding part 11j of the block 11a constituting a front end part and inserted between adjacent cervical vertebral bodies 21 and 21 from the front side. Subsequently, the falling preventive part 11h constituting a rear end part of the handle 11e is rotated and removed from the shaft 11c. The spacer 11g constituting an intermediate part is drawn out. Thereafter, the falling preventive part 11h is attached again by screwing as shown in Fig. 21 (b). Subsequently, while fixing the falling preventive part 11h to keep the shaft 11c stationary, the handle main body 11f is drawn out until it comes into contact with the falling preventive part 11h to thereby draw out the block 11a constituting a front end part from the space between the upper and lower vertebral bodies 21 and 21 as shown in Fig. 21 (c). Even when the block 11a is drawn out in this manner, the artificial intervertebral disk 35 is prevented from being drawn out of the space between the vertebrae because it is supported by the artificial-intervertebral-disk support piece 11d on the front end of the shaft 11c so as not to move forward from the intervertebral space. Thus, the artificial intervertebral disk 35 can be disposed in a proper position between the vertebrae without fail.

As shown in Figs. 7 and 9, the artificial intervertebral disk 3 to be inserted between vertebrae of the vertebral column (especially lumbar vertebral column) with the artificial-intervertebral-disk insertion jig 10 described above is a circular-arc-shaped artificial intervertebral disk obtained by superposing plates 3b and 3b which comprise a biodegradable and bioabsorbable polymer containing bioactive bioceramic particles and have bone conductivity respectively on the upper and lower sides of a core material 3a comprising a structure made up of organic fibers and disposing two or more pins 3c for fixing (three pins in Fig. 7) made of a biodegradable and bioabsorbable polymer so that the pins 3c vertically extend through the core material 3a and the plates 3b and 3b and the tips 3d of the pins protrude from the upper and lower surfaces. The core material 3a comprises a structure which is either a three-dimensional woven structure or knit structure made of organic fibers or a structure comprising a combination of the woven structure and the knit structure. It is a core material having almost the same mechanical strength and flexibility as intervertebral disks of the living body and the deformation properties thereof are highly biomimetic (biomimetic). The structure of this core material 1 is the same as the structure described in Japanese Patent Application No. 1994-254515, which was filed by the applicant. When the geometry of this core material is expressed in terms of the number of dimensions and the number of directions of fiber arrangement is expressed in terms of the number of axes, then the structure preferably is a multiaxis three-dimensional structure with three or more axes.

The three-axis three-dimensional structure is a structure made up of three-dimensionally arranged fibers extending in three axial directions, i.e., length, breadth, and vertical directions. A typical shape of this structure is a thick bulk shape (platy or block shape) such as the core material 3a. This kind of three-axis three-dimensional structures are classified, according to structure differences, into orthogonal structure, non-orthogonal structure, leno structure, cylindrical structure, etc. A multiaxis three-dimensional structure with four or more axes has an advantage that the strength isotropy of the structure can be improved by arranging fibers in directions along 4, 5, 6, 7, 9, 11 axes, etc. By selecting these, a core material 3a which is more biomimetic and more akin to intervertebral disks of the living body can be obtained.

The core material 3a comprising the structure described above preferably has an internal porosity in the range of 20-90%. In case where the internal porosity thereof is lower than 20%, this core material 3a is too dense and is impaired in flexibility and deformability. This material is hence unsatisfactory as the core material of an artificial intervertebral disk. In case where the internal porosity thereof exceeds 90%, this core material 3a is reduced in compression strength and shape retention. This material also is hence unsuitable for use as the core material of an artificial intervertebral disk.

As the organic fibers for constituting the core material 3a are preferably used bioinert synthetic resin fibers such as, e.g., fibers of polyethylene, polypropylene, polytetrafluoroethylene, or the like and coated fibers obtained by coating organic core fibers with any of these bioinert resins to impart bioinertness. In particular, coated fibers having a diameter of about 0.2-0.5 mm obtained by coating core fibers of ultrahigh-molecular polyethylene with linear low-density polyethylene are optimal fibers from the standpoints of strength, hardness, elasticity, suitability for weaving/knitting, etc. Besides these, fibers having bioactivity (e.g., having bone conductivity or bone inductivity) can be selected.

A further explanation on the structure of the core material 3a is omitted because the structure is disclosed in detail in Japanese Patent Application No. 1994-254515, which was cited above.

The plates 3b and 3b superposed respectively on the upper and lower sides of the core material 3a are nonporous plates made of a biodegradable and bioabsorbable polymer containing bioactive bioceramic particles. Use may be made of one obtained by melt-molding the polymer or one obtained by subjecting the melt-molded object to cold forging (at a temperature which is not lower than the glass transition temperature of the polymer and is lower than the melting temperature thereof). Even when such plates 3b and 3b are superposed respectively on both sides of the core material 3a, a living-body bone penetrates from lateral sides along the upper and lower surfaces of each plate through minute gaps among the three members due to bone conductivity. The bone penetrates into the space between each vertebral body and the corresponding plate and the space between each plate and the surface-treated core material 3a and grows there to bond at the interfaces among the three members.

The latter plates, i.e., forged plates, may be ones obtained by forging the melt-molded object once or may be ones obtained by forging two or more times. In particular, however, plates obtained by subjecting an object which as forged once to forging once again in a changed machine direction have an advantage that they are less apt to deteriorate mechanically or break even when repeatedly deformed by external forces, because the thus-forged plates have a structure in which molecular chains or crystals of the polymer have been oriented along many reference axes randomly different in direction, or a structure made up of many clusters of these which have many reference axes randomly different, or a structure in which molecular chains, crystals, and clusters are oriented in three-dimensional directions. Consequently, when an artificial intervertebral disk 3 comprising a core material 3a and, superposed on each side thereof, such a plate 3b which has undergone forging twice is inserted between vertebral bodies 20 and 20 with the insertion jig 10 described above, then the plates 3b do not suffer mechanical deterioration, breakage, or the like until the plates 3b are mostly degraded and absorbed, even when the plates 3b are repeatedly deformed together with the core material 3a by the sandwiching pressure of the upper and lower vertebral bodies 20 and 20. Furthermore, even the plates whichhave undergone forging once have improvedmechanical strength and less susceptibility to breakage as compared with plates obtained through mere melt molding, because the plates have been densified by compression and come to have a structure in which molecular chains or crystals of the polymer are oriented obliquely to one reference axis or reference plane or a structure in which the molecular chains or crystals are oriented along many axes as described above.

Preferred examples of the biodegradable and bioabsorbable polymer to be used as a material of the plates 3b include poly(lactic acid)s, such as poly(L-lactic acid), poly (D-lactic acid), and poly(D,L-lactic acid), and copolymers of any of L-lactide, D-lactide, and DL-lactide with glycolide, caprolactone, dioxanone, ethylene oxide, or propylene oxide. These may be used alone or as a mixture of two or more thereof. Of these polymers, the poly (lactic acid) s preferably are ones having a viscosity-average molecular weight of about 50,000-500,000 from the standpoints of the rate of degradation/absorption of the plates 3b which is balanced with the growth of bone tissues, the degradation/absorption period (1-odd year) and the mechanical strength which enables the plates 3b to withstand the sandwichingpressure of the vertebral bodies, etc.

As the bioceramic particles to be incorporated in the biodegradable and bioabsorbable polymer, use is made of ones having bioactivity and having satisfactory bone conductivity and satisfactory biocompatibility, such as uncalcined or unsintered particles of hydroxyapatite, dicalcium phosphate, tricalcium phosphate, tetracalcium phosphate, octacalcium phosphate, calcite, ceravital, diopside, or natural coral. Also usable are ones obtained by adhering an alkaline inorganic compound or a basic organic substance to the surface of these particles. Preferred of these are in the living body wholly absorbable bioceramic particles which are wholly absorbed in the living body and completely replaced by bone tissues. In particular, uncalcined or unsintered hydroxyapatite, tricalcium phosphate, and octacalcium phosphate are optimal because they have exceedingly high activity and excellent bone conductivity, are less harmful, and are absorbed by the living body in a short period. The particles of any of these bioceramics to be used have an average particle diameter of 10 µm or smaller, preferably about 0.2-5 µm.

The content of the bioceramic particles is preferably regulated to 25-60% by mass. Contents thereof exceeding 60% by mass are disadvantageous because the plates 2 become brittle and are hence apt to break due to the sandwiching pressure of vertebral bodies. Contents thereof lower than 25% by mass are disadvantageous because the conductive growth of bone tissues becomes slow and, hence, a prolonged period is required for the plates 3b to be replaced by bone tissues. The content of the bioceramic is more preferably 30-50% by mass.

Besides the bioceramic particles, various cytokines and drugs having bone inductivity may be incorporated into the plates 3b in a suitable amount. In this case, there is an advantage that the growth of and replacement by bone tissues, which occur with the degradation/absorption of the plates 3b, are considerably accelerated and the core material 3a is directly bonded to vertebral bodies 20 in an early stage. A bone induction factor (bone morphogenetic protein) may also be incorporated into the plates 3b. This incorporation is more effective in bonding/integration because bone induction occurs. Furthermore, the bioceramic particles, cytokine, and bone induction factor may be applied by spraying to the surfaces of the core material 3a. In this case, there is an advantage that since the surfaces of the core material 3a become bioactive and bone tissues which have conductively grown bond to the activated surfaces, direct bonding between vertebral bodies 20 and the core material 3a is accomplished in a relatively short period while maintaining strength.

It is preferred that fine recesses and protrusions be formed on both sides of the plates 3b. Such recesses and protrusions bring about the following advantages. When the artificial intervertebral disk 3 is inserted between vertebral bodies 20 and 20; the protrusions of the recesses and protrusions formed on the front side of each plate 3b bite into the terminal plate of the vertebral body 20 to prevent the artificial intervertebral disk 3 from suffering positional shifting/falling off. Furthermore, the recesses and protrusions on the front side are effective in bonding because they considerably increase the area of contact with the vertebral body 20. On the other hand, the protrusions of the recesses and protrusions formed on the back side of each plate 3b bite into the core material 3a to prevent the plate 3b and the core material 3a from suffering relative positional shifting. Consequently, in the case where recesses and protrusions are formed on both sides of each plate 3b, the pins 3c may be omitted.

The fine recesses and protrusions may have random shapes. However, the recesses and protrusions preferably are ones in which the protrusions are many fine protrusions of a pyramid shape (e.g., a regular quadrangular pyramid shape in which each side of the square bottom face has a length of about 0.6 mm and the pyramid height is about 0.3 mm) arranged closely so that each protrusion is arranged without any gap in an anteroposterior direction and in a horizontal direction. The formation of such recesses and protrusions has an advantage that since the pyramidal protrusions are apt to bite into the terminal plate of the vertebral body 20 and into the core material 3a, the positional shifting/falling off of the artificial intervertebral disk 3 and the relative positional shifting of each plate 3b and the core material 3a can be prevented with higher certainty. Incidentally, pyramidal or conical projections which have a larger height than these fine recesses and protrusions (i.e., projections having a height of about 0.5-1.5 mm) may be formed on the front side of each plate 3b together with the fine recesses and protrusions. This constitution has an advantage that since the larger projections deeply bite into the terminal plate of the vertebral body 20, the positional shifting/falling off of the artificial intervertebral disk 3 can be prevented with even higher certainty.

The thickness of each plate 3b is desirably regulated to a value in the range of 0.3-1.2 mm, especially preferably to about 1 mm. In the case where fine recesses and protrusions are formed on both sides of each plate 3b, it is preferred that the thickness of the thinnest parts (the distance between the recess bottom on one side and the recess bottom on the other side) be regulated to 0.3 mm or larger and the thickness of the thickest parts (the distance between the protrusion top on one side and the protrusion top on the other side) be regulated to 1.2 mm or smaller. The plates 3b having such specific values of thickness have advantages that they have a strength which enables the plates 3b to withstand the sandwiching pressure of the upper and lower vertebral bodies 20 and 20, and that the plates 3b are degraded and absorbed at a rate balanced with the growth of bone tissues and are completely replaced by bone tissues to complete tenacious bonding to the vertebral bodies 20 in 1-odd year. In case where the thickness of each plate 3b (thickness of the thinnest parts when recesses and protrusions have been formed) is smaller than 0.3 mm, there is a possibility that the plates 3b might have insufficient strength and break due to the sandwiching pressure of the vertebral bodies 20 and 20. On the other hand, in case where the thickness of each plate 3b (thickness of the thickest parts when recesses and protrusions have been formed) is larger than 1.2 mm, a trouble arises that the time period required for the degradation/absorption of the plates 3b is prolonged and replacement by bone tissues becomes slow.

Subjecting both sides of each plate 3b to an oxidation treatment such as corona discharge, plasma treatment, or hydrogen peroxide treatment is preferred because the wettability of the bioceramic particles exposed on the surfaces is improved and the penetration and growth of bone cells to be proliferated occur effectively.

The pins 3c which vertically extend through the core material 3a and the two plates 3b and 3b disposed respectively on both sides of the core material 3a preferably are pins which are made of the lactic acid polymer described above and the strength of which has been heightened by orienting polymer molecules or crystals through forging conducted once or twice or through stretching. The tips of each pin 3c which protrude from the plates 3b and 3b have been formed in a conical shape having a height of about 0.3-2 mm so that when this artificial intervertebral disk 3 is inserted between vertebral bodies 20 and 20, the tips of each pin 3c deeply bite into the terminal plates of the vertebral bodies 20 and 20 to thereby prevent the positional shifting/falling off of the artificial intervertebral disk 3 without fail. The artificial intervertebral disk 3 may have only one pin 3c. However, disposition of only one pin 3c has a drawback that although this artificial intervertebral disk 3 may be prevented from suffering lateral-direction positional shifting, it cannot be prevented from rotating. It is therefore desirable to dispose two or more pins. Preferably, three pins extending through the artificial intervertebral disk 3 are disposed at a given interval along the center line of the disk 3 as shown in Fig. 7. This disposition of three pins 3c has an advantage that the artificial intervertebral disk 3 can be stably attached to a position between the upper and lower vertebral bodies 20 and 20 due to three-point support. With respect to the thickness of the pins 3c, the diameter thereof is desirably regulated to about 0.5-3 mm, preferably about 1 mm, so as to prevent the pins 3c frombeingbrokenor damaged by the sandwiching pressure of the vertebral bodies 20 and 20.

It is preferred that the bioceramic particles described above and any of various cytokines, drugs, bone induction factors, and the like should be incorporated also into the pins 3c in a suitable amount. In some cases, the pins 3c may be united with the plates 3b and 3b by adhesive bonding, fusion bonding, etc. Furthermore, use may be made of a method in which each pin 3a is divided into an upper part and a lower part and these upper and lower pins are disposed so that the upper tip of the upper pin and the lower tip of the lower pin protrude respectively from the upper and lower plates 3b and 3b.

When the artificial intervertebral disk 3 having the constitution described above is inserted between adjacent vertebral (especially lumbar vertebral) bodies 20 and 20 with the artificial-intervertebral-disk insertion jig 10 described above, the tips 3d and 3d of each pin which protrude from the front sides of the plates 3b and 3b of the artificial intervertebral disk 3 bite into the terminal plates of the vertebral bodies 20 and 20 as shown in Fig. 12. As a result, the artificial intervertebral disk 3 is sandwiched between the vertebral bodies 20 and 20 and fixed thereto without undergoing positional shifting/falling off. The core material 3a, which comprises a structure made up of organic fibers and having almost the same mechanical strength and flexibility as intervertebral disks of the living body, biomimetically deforms to sufficiently function as an intervertebral disk. Even when the core material 3a thus undergoes biomimetic deformations repeatedly under the high sandwiching pressure of the upper and lower vertebral bodies 20 and 20, the plates 3b and 3b of the artificial intervertebral disk 3 hardly wear because they are not brittle. Especially when the plates 3b and 3b each are the forging described above, repetitions of deformations hardly result in mechanical deterioration or breakage. Upon contact with a body fluid, hydrolysis and absorption proceed from the surfaces of these plates 3b and 3b. With this hydrolysis/absorption, bone tissues grow conductively toward inner parts of the plates 3b and 3b due to the bone conductivity of the bioceramic particles. Since the rate of hydrolysis/absorption of each plate 3b differs little from the rate of growth of bone tissues, the whole plates 3b and 3b are finally replaced in 1-odd year by the bone tissues which grow with the degradation/absorption of the plates 3b and 3b. Thus, the artificial intervertebral disk 3 directly bonds to the vertebral bodies 20 and 20 and is tenaciously fixed. Consequently, the strength of fixing to the vertebral bodies 20 and 20 is improved.

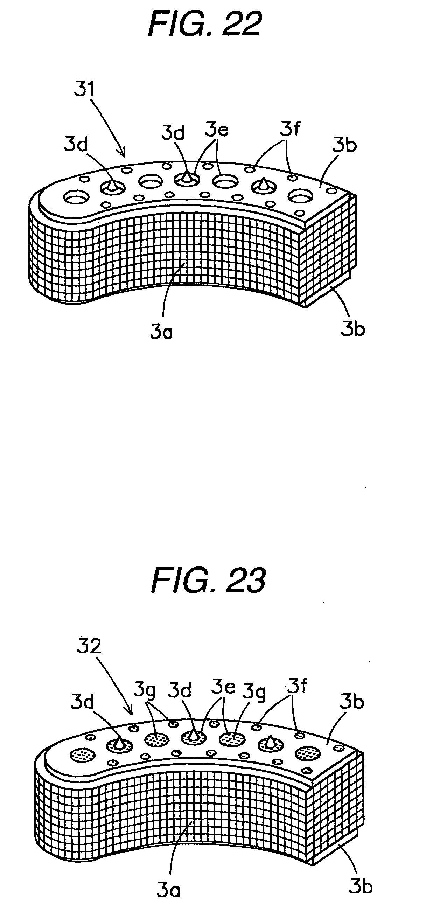

The artificial intervertebral disk 31 shown in Fig. 22 is one obtained from the artificial intervertebral disk 3 described above by forming large perforations 3e in the artificial intervertebral disk 3 at an interval along the central lines of the plates 3b and 3b, forming small perforations in peripheral parts of the plates 3b and 3b at an interval, and inserting pins into some of the perforations 3e so as to make the tips 3d of the pins protrude. Consequently, the core material 3a and pins 3c of this artificial intervertebral disk 31 are the same as those in the artificial intervertebral disk 3 described above. The material and others of the plates 3b also are the same. Consequently, an explanation on these is omitted.

It is preferred that large and small perforations 3e and 3f should be formed in each plate 3b of this artificial intervertebral disk 31 so as to result in a rate of openings of 15-60%. The plate 3b thus regulated so as to have a rate of openings of 15-60% has advantages that it has a strength which enables the plate 3b to withstand the sandwiching pressure of the upper and lower vertebral bodies 20 and 20 and that the rate of degradation/absorption of the whole plate is moderate and balanced with the rate of growth of bone tissues to thereby attain complete replacement by bone tissues and tenacious bonding to the vertebral body 20. Rates of openings higher than 60% are undesirable because the plate 3b has a reduced strength. Rates of openings lower than 15% are undesirable because the time period required for the degradation/absorption of the plate 2 tends to be prolonged and replacement by bone tissues tends to become slow.

The diameters of the large and small perforations 3e and 3f are not particularly limited. However, it is preferred to suitably regulate the diameters of the large perforations 3e and small perforations 3f in the range of 0.5-5 mm. In case where the diameter of the large perforations 3e exceeds 5 mm, this is undesirable because the perforations 3e are less apt to be completely filled with growing bone tissues, resulting in a possibility that it might be difficult to grow bone tissues over the whole surfaces of the core material 1.

This artificial intervertebral disk 31 has large perforations 3e formed along the center line of each plate 3b and small perforations 3f formed in a peripheral part of each plate 3b. However, it is possible to form large and small perforations 3e and 3f in random arrangement so as to be almost evenly dispersed. It is also possible to dispersedly form perforations having the same diameter, in place of forming large perforations separately from small perforations. The shape of the perforations 3e and 3f is not limited to circle, and the perforations may be formed in any desired shape selected from ellipses, elongated circles, quadrilaterals, other polygons, irregular shapes, and the like. Consequently, quadrilateral perforations of the same size may, for example, be formed in lattice arrangement to constitute a net-form plate 3b.

Furthermore, in this artificial intervertebral disk 31, it is preferred that a yarn (not shown in the figure) should be passed through the small perforations 3f formed in a peripheral part of each plate 3b to sew the plate 3b to the core material 3a so as to cover the periphery of the plate 3b, whereby the plate 3b is fixed so as to be prevented from suffering relative positional shifting or separation from the core material 3a. A suitable yarn is one comprising a bioinert fiber, biodegradable fiber, or the like. As the former fiber, i.e., bioinert one, maybe used the organic fiber for use in constituting the core material 3a. As the latter fiber, i.e., biodegradable one, may be used a fiber made of the lactic acid polymer described above. Such yarns to be used are yarns (monofilaments) which have a thickness of about 0.2-0.3 mm and which preferably have been uniaxially stretched and have a high tensile strength.

When the artificial intervertebral disk 31 described above is inserted between vertebral (especially lumbar vertebral) bodies 20 and 20 with the artificial-intervertebral-disk insertion jig 10, the following advantage is brought about besides the same effects as those produced with the artificial intervertebral disk 3 described above. After the insertion, a body fluid passes through the perforations 3e and 3f of each plate 3b and reaches the back side of the plate 3b to cause degradation/absorption to proceed also on the back side of the plate 3b. Because of this, bone tissues can grow on both sides of each plate 3b to attain direct bonding to the vertebral bodies 20 and 20 in an early stage.

The artificial intervertebral disk 32 shown in Fig. 23 is one obtained from the artificial intervertebral disk 31 described above by filling the perforations 3e and 3f of each plate 3b with a biodegradable and bioabsorbable material 3g having bone conductivity and/or bone inductivity and excellent bioactivity and undergoing degradation in the living body at a higher rate than the plate 3b.

The biodegradable and bioabsorbable material 3g most preferably is a porous biodegradable and bioabsorbable polymer which has interconnectedpores inside and contains thebioceramic particles having bone conductivity and/or any of various cytokines, drugs, and bone induction factors (BMF) each having bone inductivity. Also preferably used is a porous or nonporous object comprising collagen and, incorporated therein, bioactive bioceramic particles or a bone induction factor. Furthermore, a nonporous obj ect comprising a biodegradable and bioabsorbable polymer containing bioceramic particles in a larger amount than in the plate 3b is also usable. The content of the bioceramic particles in these porous or nonporous objects is preferably regulated to 60-90% by mass. The content of the cytokine, drug, or bone induction factor having bone inductivity may be a suitable amount.

The porous object to be used as the biodegradable and bioabsorbable material 3g is not required to have high strength and is required to degrade more rapidly than the plate 3b and be speedily replaced by bone tissues which grow conductively and/or inductively. Because of this, a suitable biodegradable and bioabsorbable polymer for use as a raw material for this porous object is one which is amorphous or is both crystalline and amorphous and which is safe, degraded relatively rapidly, and not so brittle. Examples thereof include poly(D,L-lactic acid), copolymers of L-lactic acid and D,L-lactic acid, copolymers of a lactic acid and glycolic acid, copolymers of a lactic cid and caprolactone, copolymers of a lactic acid and ethylene glycol, and copolymers of a lactic acid and p-dioxanone. These may be used alone or as a mixture of two or more thereof. From the standpoints of the ease of porous-object formation, period of biodegradation/bioabsorption, etc., these polymers to be used preferably have a viscosity-average molecular weight of about 50,000-1,000,000.

The porous object of the polymer desirably is one which has a porosity of 50-90% (preferably 60-80%) and in which interconnected pores account for 50-90% (preferably 70-90%) of all pores and the interconnected pores have a pore diameter of about 100-400 µm (preferably 150-350 µm), when physical strength, penetration and stabilization of osteoblast, etc. are taken into account. In case where the porosity exceeds 90% and the pore diameter exceeds 400 µm, the porous object has reduced physical strength and is brittle. On the other hand, when the porosity is lower than 50%, the proportion of interconnected pores is lower than 50% based on all pores, and the pore diameter thereof is smaller than 100 µm, then the penetration of a body fluid or osteoblast becomes difficult and the hydrolysis of the porous object and the growth of bone tissues become slow. In this case, the time period required for the porous object to be replaced by bone tissues is hence prolonged.

Incidentally, methods for producing the porous object are not particularly limited and it may be produced in any method. For example, the porous object can be produced by a method which comprises: dissolving the biodegradable and bioabsorbable polymer in a volatile solvent andmixingbioceramic particles and other ingredients therewith to prepare a suspension; forming this suspension into fibers by, e.g., spraying to obtain a fibrous mass made up of intertwined fibers; packing the fibrous mass into the perforations 3e and 3f of each plate 3b which has not been superposed; heating this plate 3b to a temperature at which the fibers are fusion-bondable to thereby partly fusion-bond the fibers to one another and obtain a porous fusion-bonded fibrous mass; and immersing this fusion-bonded fibrous mass in a volatile solvent together with the plate 3b to convert the fibrous mass into a porous object.

The biodegradable and bioabsorbable material 3g with which the perforations are filled is degraded more rapidly than each plate 3b, and bone tissues rapidly grow conductively and/or inductively due to the bone conductivity of the bioceramic particles contained and the bone inductivity of the cytokine, drug, or bone induction factor to replace the biodegradable and bioabsorbable material 3g in the perforations 3e and 3f in an early stage.
On the other hand, the plate 3b is degraded more slowly than the biodegradable and bioabsorbable material 3g and retains sufficient strength until the biodegradable and bioabsorbable material 3g in the perforations 3e and 3f is replaced by bone tissues to some degree. Thereafter, the plate 3b is wholly replaced by bone tissues and finally attains complete and tenacious bonding to the vertebral body 20.

Although all the perforations 3e and 3f of the artificial intervertebral disk 32 shown in Fig. 23 are filled with a biodegradable and bioabsorbable material 3g, it is possible to fill part of the perforations, e.g., only the large perforations 3e, with the material 3g.

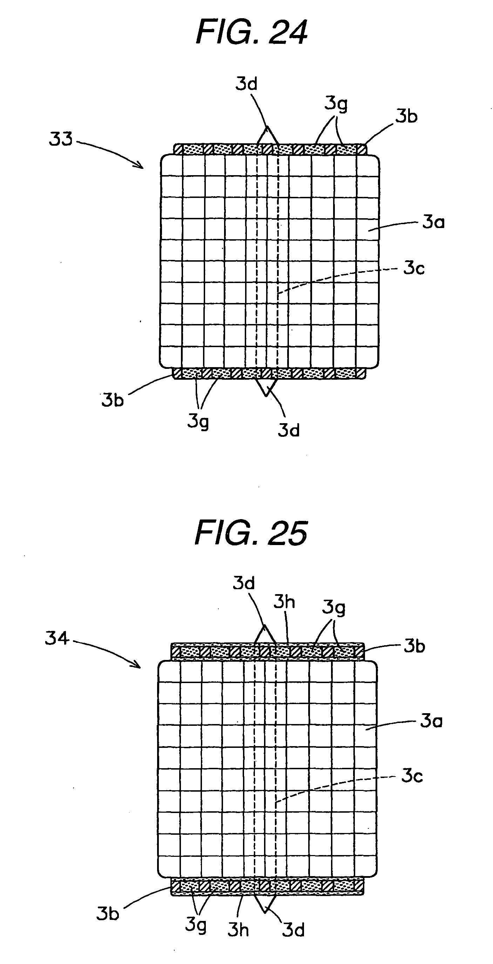

The artificial intervertebral disk 33 shown in Fig. 24 comprises a core material 3a and plates 3a superposed respectively on the upper and lower sides of the core material 3a, wherein each plate 3b has been formed in a net form having square openings and these square openings are filled with the biodegradable and bioabsorbable material 3g having bone conductivity and/or bone inductivity described above. The other constitutions of this artificial intervertebral disk 33 are the same as those of the artificial intervertebral disk 3 described above. Consequently, like members are designated by like numerals in Fig. 24, and an explanation is omitted.

This artificial intervertebral disk 33 functions like the artificial intervertebral disk 32 described above. Namely, the biodegradable and bioabsorbable material 3g in the square openings in each plate 3b is rapidly replaced by bone tissues and the net-form plate 3b retains strength until the biodegradable and bioabsorbable material 3g is replaced by bone tissues to some degree. Finally, the biodegradable and bioabsorbable material 3g is wholly replaced by bone tissues and attains complete and tenacious bonding to the vertebral body 20.

The artificial intervertebral disk 34 shown in Fig. 25 is one obtained from the artificial intervertebral disk 33 described above by forming covering layers 3h and 3h made of the biodegradable and bioabsorbable material having bone conductivity and/or bone inductivity described above respectively on the obverse and reverse sides of each plate 3b. The other constitutions of this artificial intervertebral disk 34 are the same as those of the artificial intervertebral disk 3 described above. Consequently, like members are designated by like numerals in Fig. 24, and an explanation is omitted.

In this artificial intervertebral disk 34, bone tissues almost evenly grow on the surfaces of each plate 3b in an early stage. Especially in the case where each covering layer 3h is a porous layer comprising the biodegradable and bioabsorbable polymer described above which contains bioceramic particles and a cytokine or the like, this covering layer 3h functions as a cushioning material and adheres to a vertebral body 20 to facilitate the penetration of osteoblast into inner parts of the porous layer. Consequently, bone tissues rapidly grow conductively and/or inductively, and bonding to the vertebral body 20 is accomplished in a short period.

Although the artificial intervertebral disk 34 shown in Fig. 25 has a covering layer 3h formed on each of the obverse and reverse sides of each plate 3b, the covering layer may be formed only on the front side of each plate 3b. Furthermore, this covering layer 3h may be formed on each of the obverse and reverse sides of or on the front side of: the plates of the artificial intervertebral disk 3 described above which havenoperforations; the plates of the artificial intervertebral disk 31 described above which have perforations formed therein; and the plates of the artificial intervertebral disk 32 described above which have perforations filled with a biodegradable and bioabsorbable material.

The artificial intervertebral disk 35 shown in Fig. 26 is a whole replacement type artificial intervertebral disk for insertion between cervical vertebrae from the front side with the insertion jig 11 described above. This artificial intervertebral disk has been formed in a shape which is square at the front and rounded at the rear, i.e., which comprises a combination of a rectangular part as a front half and a semi-circular part as a rear half. This artificial intervertebral disk 35 differs in shape from the partial replacement type artificial intervertebral disk 3 described above for insertion between vertebrae (especially lumbar vertebrae). However, it has the same constitution as the artificial intervertebral disk 3, and is obtained by superposing plates 3b and 3b respectively on both sides of a core material 3a and disposing two pins 3c so that they extend vertically through the core material 3a and the plates 3b and 3b and the tips 3d of each pin 3c protrude from the surfaces of the plates 3b.

The artificial intervertebral disk 36 shown in Fig. 27 is a partial replacement type artificial intervertebral disk having a shape obtained by dividing the artificial intervertebral disk 35 into a right and left part. Although different in shape, this artificial intervertebral disk 36 has the same constitution as the artificial intervertebral disk 35.

These artificial intervertebral disks 35 and 36 are intended to be inserted between cervical vertebrae. However, in the case of insertion between vertebrae of the vertebral column (especially the lumbar vertebral column), these artificial intervertebral disks each are modified so as to have an almost similar shape and larger dimensions. It is a matter of course that the shapes and sizes of these artificial intervertebral disks may be suitably modified according to insertion parts.

In these artificial intervertebral disks 35 and 36 also, it is preferred to make the following modifications as described above: to employ plates 3b which are forgings; to form fine recesses and protrusions on both sides of each plate 3b; to form projections on the front side of each plate 3b; to form perforations in each plate 3b so as to result in a rate of openings in the plate 3b of 15-60%; to fill the perforations 3e and 3f with a biodegradable and bioabsorbable material 3g which is degraded rapidly and has bone conductivity and/or bone inductivity; to form a covering layer 3h made of the biodegradable and bioabsorbable material 3g on the front side of or on each of the obverse and reverse sides of each plate 3b; and to sew the periphery of each plate 3b to the core with a yarn.

Those artificial intervertebral disks have shapes which fit the insertion jigs of the invention as described above. However, use of a different insertion jig requires the artificial intervertebral disks to have another shape. The artificial intervertebral disk of the invention can hence be used with other insertion jigs of various kinds and be used also in operations in which no insertion jigisused. Furthermore, although insertion from the back side was mainly described, operation techniques are not limited and the artificial intervertebral disk of the invention can be inserted from the front side or a lateral side.

### INDUSTRIAL APPLICABILITY

With the artificial-intervertebral-disk insertion jigs and jig set of the invention, an artificial intervertebral disk can be easily and precisely inserted between vertebrae of the vertebral (especially lumbar vertebral) column or cervical vertebral column from the back side or front side (which is essential especially to whole replacement). The insertion jigs and jig set are hence suitable for the current trend toward low-invasion operation techniques. In addition, since the artificial intervertebral disk of the invention to be inserted employs a fibrous structure as a core material, has flexible and nearly ideal deformation properties, and can be bonded and fixed to vertebral bodies at a high force, it is exceedingly effective in the treatment of intervertebral diskhernia, serious intervertebral disk damages, and the like.

## Claims

1. An artificial-intervertebral-disk insertion jig, which comprises an upper jig and a lower jig, as a pair of jigs, which have been fitted to each other so as not to separate from each other in vertical directions and as to be capable of sliding against each other in back-and-forth directions, the upper jig and the lower jig having an upper holding part and a lower holding part which serve to hold an artificial intervertebral disk and have been formed as front parts of the upper jig and lower jig.

2. The artificial-intervertebral-disk insertion jig according to claim 1, wherein either of the upper jig and the lower jig has a ridge having a sectional shape with an expanded top and the other has a groove having a sectional shape with an expanded bottom so as to correspond to the ridge, and the ridge and the groove have been fitted to each other, whereby the upper jig and the lower jig have been fitted to each other so as not to separate from each other in vertical directions and as to be capable of sliding against each other in back-and-forth directions.

3. The artificial-intervertebral-disk insertion jig according to claim 1 or 2, wherein the upper holding part of the upper jig and the lower holding part of the lower jig respectively have slots for holding the tips of each pin which protrude respectively from the upper and lower sides of the artificial intervertebral disk.

4. The artificial-intervertebral-disk insertion jig according to any one of claims 1 to 3, wherein the front end of the upper holding part of the upper jig and the front end of the lower holding part of the lower jig have been formed in a semicircular shape or semi-elliptic shape.

5. The artificial-intervertebral-disk insertion jig according to any one of claims 1 to 4, wherein the upper jig and the lower jig are made of ultrahigh-molecular polyethylene.

6. The artificial-intervertebral-disk insertion jig according to any one of claims 1 to 5, wherein the upper jig and the lower jig are circular-arc-shaped bent jigs having the same radius of curvature and have been fitted to each other so as to be capable of sliding against each other in the circular-arc directions which are the back-and-forth directions for the jigs.

7. The artificial-intervertebral-disk insertion jig according to any one claims 1 to 6, wherein either of the upper jig and the lower jig has a stopper which inhibits the jig from sliding forward from a position in which the upper jig overlies the lower jig.

8. The artificial-intervertebral-disk insertion jig according to claim 7, which has a handle attached to the rear end of the upper jig through a shaft.

9. An artificial-intervertebral-disk insertion jig, which comprises a block having an upper holding part and a lower holding part which serve to hold an artificial intervertebral disk, a pipe attached to a rear part of the block, and a shaft which has been inserted in the pipe in a freely slidable manner and has an artificial-intervertebral-disk support piece attached to the front end of the shaft, the support piece being located in a recessed space formed at the back of the space between the upper holding part and lower holding part of the block.

10. A jig set which comprises the artificial-intervertebral-disk insertion jig of any one of claims 1 to 8 and an insertion guide jig having a guide hole, wherein the insertion guide jig, when the insertion jig is inserted in the guide hole, guides the insertion jig so that the artificial intervertebral disk held by the upper holding part and lower holding part reaches a given position between vertebrae.

11. An artificial intervertebral disk which comprises: a core material comprising a structure which is either amultiaxis three-dimensional woven structure or knit structure made of organic fibers arranged along three or more axes or a structure comprising a combination of the woven structure and the knit structure; and plates superposed respectively on the upper and lower sides of the core material, the plates having bone conductivity and being made of a biodegradable and bioabsorbable polymer containing bioactive bioceramic particles.

12. The artificial intervertebral disk according to claim 11, wherein the plates each is a forging of a biodegradable and bioabsorbable polymer containing bioactive bioceramic particles.

13. The artificial intervertebral disk according to claim 11 or 12, wherein the plates have many perforations formed therein.

14. The artificial intervertebral disk according to claim 13, wherein the plates have a rate of openings of 15-60%.

15. The artificial intervertebral disk according to claim 13 or 14, wherein the perforations of the plates are partly or wholly filled with a biodegradable and bioabsorbable material having bone conductivity and/or bone inductivity and excellent bioactivity and undergoing degradation in the living body at a higher rate than the plates.

16. The artificial intervertebral disk according to claim 15, wherein the plates each has a covering layer formed on the front side thereof or on each of the obverse and reverse sides thereof, the covering layer being made of a biodegradable and bioabsorbable material having bone conductivity and/or bone inductivity and excellent bioactivity and undergoing degradation in the living body at a higher rate than the plates.

17. The artificial intervertebral disk according to claim 15 or 16, wherein the biodegradable and bioabsorbable material is a porous obj ect of a biodegradable and bioabsorbable polymer, the porous object having interconnected pores inside and containing bioceramic particles having bone conductivity and/or any of a cytokine, a drug, and a bone induction factor each having bone inductivity.

18. The artificial intervertebral disk according to claim 15 or 16, wherein the biodegradable and bioabsorbable material is one obtained by incorporating bioactive bioceramic particles or a bone induction factor into collagen.

19. The artificial intervertebral disk of any one of claims 11 to 15, wherein the plates each has fine recesses and protrusions formed on each side thereof.

20. The artificial intervertebral disk according to anyone of claims 11 to 15, wherein the plates each has projections formed on the front side thereof.

21. The artificial intervertebral disk according to any one of claims 11 to 20, which has at least one biodegradable and bioabsorbable pin extending through the core material and the plates, the tips of thepinprotruding from the plate surfaces.

22. The artificial intervertebral disk according to any one of claims 11 to 20, wherein the periphery of each plate has been sewed to the core material with a yarn.
